# EUROPEAN PATENT APPLICATION

(11) **EP 1 870 414 A1**
(43) Date of publication of application: **26.12.2007**
(21) Application number: 06730282.8
(22) Date of filing: 28.03.2006
(51) Int. Cl.: C07D 495/04, A61K 31/4365, A61K 31/4709, A61P 35/00, A61P 43/00, C07D 401/12, C07D 471/04

(54) **THIENOPYRIDINE DERIVATIVE, OR QUINOLINE DERIVATIVE, OR QUINAZOLINE DERIVATIVE, HAVING C-MET AUTOPHOSPHORYLATION INHIBITING POTENCY**

(30) Priority: 28.03.2005 JP 2005092881
(71) Applicant: Kirin Pharma Kabushiki Kaisha, Tokyo 150-8011 (JP)
(72) Inventor: KUBO, Kazuo c/o Discovery Research Laboratories, Takasaki-shi Gunma, 3701295 (JP); FURUTA, Takayuki c/o Discovery Research Laboratories, Takasaki-shi Gunma, 3701295 (JP); OSAWA, Tatsushi c/o Discovery Research Laboratories, Takasaki-shi Gunma, 3701295 (JP); MIWA, Atsushi c/o Discovery Research Laboratories, Takasaki-shi Gunma, 3701295 (JP)
(74) Representative: HOFFMANN EITLE
(86) International application number: PCT/JP2006/306334
(87) International publication number: WO 2006/104161

(57) **Abstract**

An objective of the present invention is to provide compounds having antitumor activity. According to the present invention, there is provided compounds represented by formula (I) and pharmaceutically acceptable salts thereof, and solvates thereof: 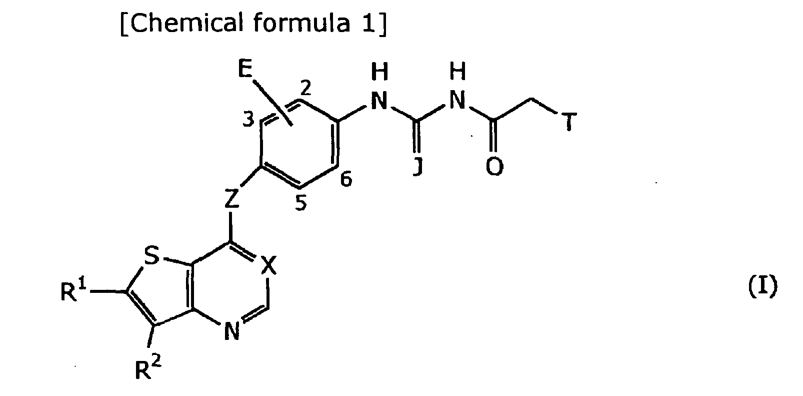 wherein R¹ represents H or a substitutable unsaturated five- or six-membered heterocyclic ring; R² represents H; X represents CH or N; Z represents O or S; E is absent or represents halogen, alkyl, or alkoxy; J represents S or O; and T represents phenyl, an unsaturated five- or six-membered heterocyclic ring, an unsaturated nine- or ten-membered bicyclic carbocyclic ring or heterocyclic ring.

## Description

### FIELD OF THE INVENTION

The present invention relates to thienopyridine derivatives, quinoline derivatives, and quinazoline derivatives having c-Met autophosphorylation inhibitory activity. More particularly, the present invention relates to thienopyridine derivatives, quinoline derivatives, and quinazoline derivatives useful for the treatment of malignant tumors.

### BACKGROUND ART

Growth factors such as epithelial growth factors, platelet-derived growth factors, insulin-like growth factors, and hepatocyte growth factors (hereinafter abbreviated to "HGF") play an important role in cell proliferation. Among others, HGF is known to be involved, as a liver regenerating factor and a kidney regenerating factor, in the regeneration of damaged liver and kidney (Oncogenesis, 3, 27 (1992)).

However, excessive expression of HGF and a receptor thereof (hereinafter abbreviated to "c-Met") is reported to be found in various tumors such as cerebral tumors, lung cancer, gastric cancer, pancreatic cancer, colon cancer, ovarian cancer, renal cell cancer, and prostatic cancer (Oncology Reports, 5, 1013 (1998)). In particular, in gastric cancer, excessive expression of c-Met and an increase in HGF level of serum mainly in scirrhus gastric cancers are reported (Int. J. Cancer, 55, 72, (1993)). Further, it is also known that HGF has angiogenesis activity due to the acceleration of the proliferation and migration of vascular endothelial cells (Circulation, 97, 381 (1998), and Clinical Cancer Res., 5, 3695, (1999)) and induces the dispersion and invasion of cells (J. Biol. Chem., 270, 27780 (1995)). For this reason, HGF-c-Met signals are considered to be involved in the proliferation, invasion, and metastasis of various cancer cells.

NK4, a partial peptide of HGF, is reported as the HGF receptor antagonist. For example, it is reported that NK4 inhibits c-Met phosphorylation of various cancer cells and, further, suppresses cell movement and cell invasion and has tumor enhancement inhibitory activity in an in-vivo cancer transplantation model probably through angiogenesis inhibitory activity (Oncogene, 17, 3045 (1998), Cancer Res., 60, 6737 (2000), British J. Cancer, 84, 864 (2001), and Int. J. Cancer, 85, 563 (2000)).

Since, however, NK4 is a peptide, the use of NK4 as a therapeutic agent requires a design regarding reliable stability in vivo, administration method and the like.

On the other hand, there is a report on low toxic compounds which have low molecular compounds having c-Met autophosphorylation inhibitory activity and, when orally administered, exhibit antitumor activity.

### SUMMARY OF THE INVENTION

The present inventors have found that a certain group of quinoline derivatives and thienopyridine derivatives have c-Met autophosphorylation inhibitory activity and, at the same time, have antitumor effect.

An object of the present invention is to provide compounds having antitumor activity.

According to a first aspect of the present invention, there is provided a compound represented by formula (I) or a pharmaceutically acceptable salt thereof, or a solvate thereof: 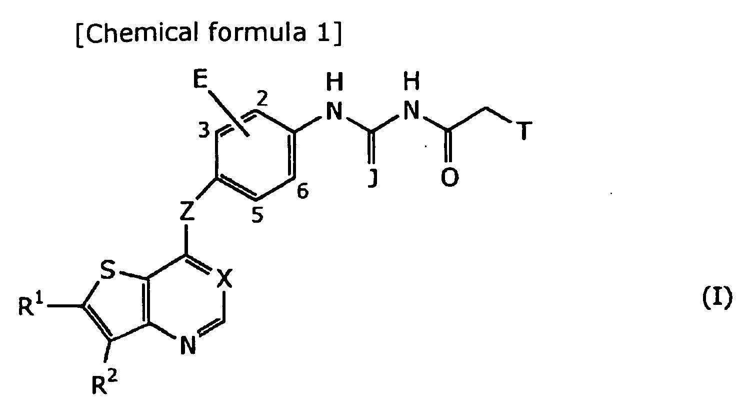 wherein
R¹ represents a hydrogen atom or an optionally substituted unsaturated five- or six-membered heterocyclic group,
R² represents a hydrogen atom,
X represents CH or N,
Z represents O or S,
E is absent or represents a substituent on the phenylene group selected from a halogen atom, C₁₋₄ alkyl, and C₁₋₄ alkoxy and the numerals represent substitutable positions,
J represents S or O, and
T represents phenyl optionally substituted by a halogen atom, C₁₋₄ alkyl, or C₁₋₄ alkoxy; an unsaturated five- or six-membered heterocyclic group optionally substituted by a halogen atom, C₁₋₄ alkyl, or C₁₋₄ alkoxy; or an unsaturated nine- or ten-membered bicyclic carbocyclic or heterocyclic group optionally substituted by a halogen atom, C₁₋₄ alkyl, or C₁₋₄ alkoxy.
   According to a second aspect of the present invention, there is provided a compound represented by formula (II) or a pharmaceutically acceptable salt thereof, or a solvate thereof: 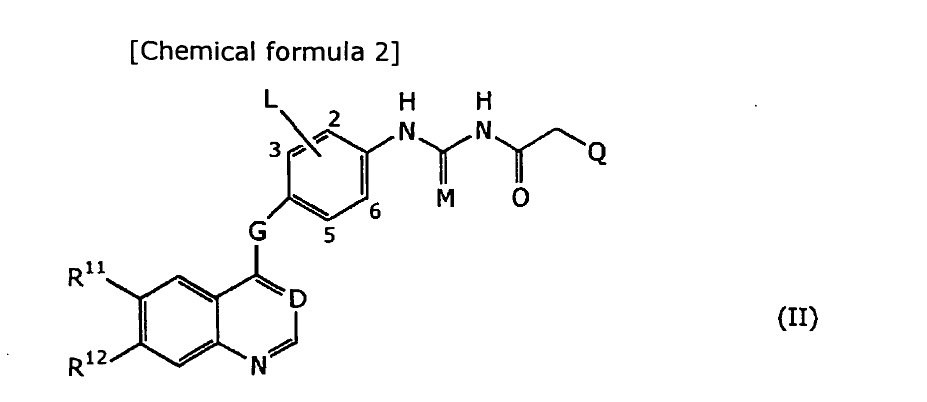
wherein
R¹¹ and R¹², which may be the same or different, represents C₁₋₄ alkoxy,
D represents CH or N,
G represents O or S,
L is absent or represents a substituent on the phenylene group selected from a halogen atom, C₁₋₄ alkyl, and C₁₋₄ alkoxy and the numerals represent substitutable positions,
M represents O or S, and
Q represents a bicyclic nine-membered unsaturated heterocyclic group optionally substituted by a halogen atom.

### BRIEF DESCRIPTION OF THE DRAWINGS

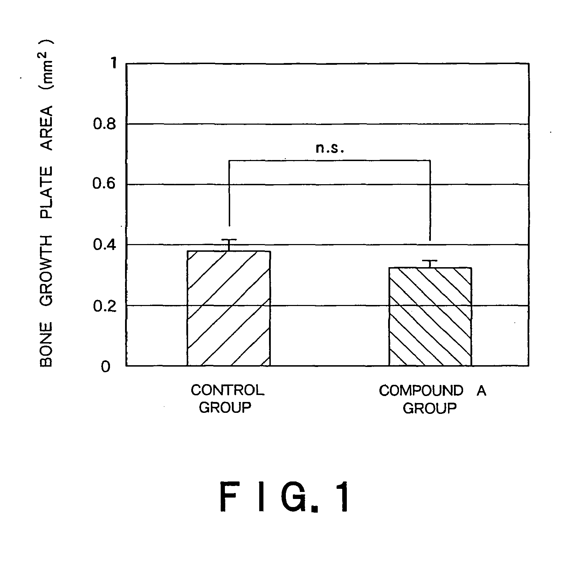

[Fig. 1] Fig. 1 is a diagram showing a bone growth plate area (mm²) for each group in Pharmacological Example 9, wherein n.s. means that there is no significant difference between the groups.

### DETAILED DESCRIPTION OF THE INVENTION

### Definition

The terms "alkyl" and "alkoxy" as used herein as a group or a part of a group respectively mean straight chain or branched chain alky and alkoxy.

Examples of C₁₋₄ alkyl include methyl, ethyl, n-propyl, isopropyl, n-butyl, i-butyl, s-butyl, and t-butyl.

Examples of C₁₋₄ alkoxy include methoxy, ethoxy, n-propoxy, i-propoxy, n-butoxy, i-butoxy, s-butoxy, and t-butoxy.

The term "halogen atom" means a fluorine, chlorine, bromine, or iodine atom.

The unsaturated five- or six-membered heterocyclic ring contains at least one hetero-atom selected from oxygen, nitrogen, and sulfur atoms. The unsaturated five- or six-membered heterocyclic ring preferably contains one or two hetero-atoms with the remaining ring-constituting atoms being carbon atoms. Examples of unsaturated five- or six-membered heterocyclic groups include thienyl and imidazolyl.

The unsaturated bicyclic nine- or ten-membered heterocyclic group contains at lest one hetero-atom selected from oxygen, nitrogen, and sulfur atoms. The unsaturated bicyclic nine- or ten-membered heterocyclic ring preferably contains one to three hetero-atoms with the remaining ring-constituting atoms being carbon atoms.

Examples of unsaturated bicyclic nine- or ten-mmbered heterocylic rings include indolyl, indazolyl, 1H-pyrrolo[2,3-b]pyridine, and 1H-pyrazolo[3,4-b]pyridine.

The term "treatment"as used herein connotes "prevention."

### Compounds in first aspect

The first aspect of the present invention relates to thienopyridine derivatives.

The unsaturated five- or six-membered heterocyclic group represented by R¹ is preferably an unsaturated five-membered heterocyclic group, more preferably imidazolyl, still more preferably 1H-imidazol-2-yl, most preferably formula (a): 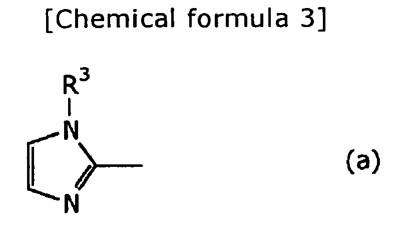 wherein R³ represents a hydrogen atom or C₁₋₄ alkyl, preferably methyl.

The substituent of the heterocyclic group represented by R¹ is preferably C₁₋₄ alkyl, more preferably methyl.

In formula (I), preferably R¹ represents an unsaturated five-membered heterocyclic group optionally substituted by C₁₋₄ alkyl, preferably methyl, more preferably imidazolyl optionally substituted by C₁₋₄ alkyl, preferably methyl, still more preferably 1H-imidazol-2-yl optionally substituted by C₁₋₄ alkyl, preferably methyl, most preferably a group of formula (a).

Preferably, X represents CH.

Preferably, Z represents O.

Preferably, E is absent or represents C₁₋₄ alkoxy, preferably methoxy, at the 2-position, or a halogen atom, preferably a chlorine atom or a fluorine atom, at the 3-position.

The unsaturated five- or six-membered heterocyclic group represented by T is preferably an unsaturated five-membered heterocyclic group, more preferably thienyl.

The unsaturated nine- or ten-membered bicyclic carbocyclic or heterocyclic group represented by T is preferably an unsaturated nine-membered bicyclic heterocyclic group, more preferably indazolyl.

Preferably, T represents phenyl optionally substituted by a halogen atom, C₁₋₄ alkyl, or C₁₋₄ alkoxy; thienyl optionally substituted by a halogen atom, C₁₋₄ alkyl, or C₁₋₄ alkoxy; or indazolyl optionally substituted by a halogen atom, C₁₋₄ alkyl, or C₁₋₄ alkoxy.

A combination of X, Z, and J is preferably a combination in which X represents CH, Z represents O, and J represents S, or a combination in which X represents CH, Z represents O, and J represents O.

Examples of preferred compounds of formula (I) include compounds in which
R¹ represents formula (a) wherein R³ represents a hydrogen atom or C₁₋₄ alkyl,
R² represents a hydrogen atom,
X represents CH,
Z represents O,
E is absent or represents a substituent on the phenylene group selected from a halogen atom, C₁₋₄ alkyl, and C₁₋₄ alkoxy and the numerals represent substitutable positions,
J represents S or O,
T represents phenyl optionally substituted by a halogen atom, C₁₋₄ alkyl, or C₁₋₄ alkoxy; an unsaturated five-membered heterocyclic group optionally substituted by a halogen atom, C₁₋₄ alkyl, or C₁₋₄ alkoxy; or an unsaturated nine-membered bicyclic heterocyclic group optionally substituted by a halogen atom, C₁₋₄ alkyl, or C₁₋₄ alkoxy.

In the above examples of preferred compounds, R³ represents C₁₋₄ alkyl, preferably methyl.

In the above examples of preferred compounds, E represents C₁₋₄ alkoxy, preferably methoxy, at the 2-position, or a halogen atom, preferably a chlorine or fluorine atom at the 3-position.

In the above examples of preferred compounds, T represents phenyl optionally represented by a halogen atom, C₁₋₄ alkyl, or C₁₋₄ alkoxy; thienyl optionally substituted by a halogen atom, C₁₋₄ alkyl, or C₁₋₄ alkoxy; and indazolyl optionally substituted by a halogen atom, C₁₋₄ alkyl, or C₁₋₄ alkoxy. Preferably, T represents phenyl optionally substituted by a halogen atom, C₁₋₄ alkyl, or C₁₋₄ alkoxy, more preferably phenyl optionally substituted by a halogen atom, or indazolyl optionally substituted by a halogen atom, C₁₋₄ alkyl, or C₁₋₄ alkoxy.

Preferred compounds among the compounds of formula (I) are as follows. The numeral within the parentheses corresponds to Example No.

(1)N-(3-chloro-4-{[2-(1-methyl-1H-2-imidazolyl)thieno[3,2-b]pyri din-7-yl]oxy}phenyl)-N-[2-(4-fluorophenyl)acetyl]thiourea;
(2)N-(3-fluoro-4-{[2-(1-methyl-1H-2-imidazolyl)thieno[3,2-b]pyrid in-7-yl]oxy}phenyl)-N-(2-phenylacetyl)urea;
(3)N-[2-(1H-1-indazolyl)acetyl]-N-(2-methoxy-4-{[2-(1-methyl-1H -2-imidazolyl)thieno[3,2-b]pyridin-7-yl]oxy}phenyl)thiourea;
(4)N-[2-(4-fluorophenyl)acetyl]-N-(4-{[2-(1-methyl-1H-2-imidazol yl)thieno[3,2-b]pyridin-7-yl]oxy}phenyl)thiourea;
(5)N-(3-fluoro-4-{[2-(1-methyl-1H-2-imidazolyl)thieno[3,2-b]pyrid in-7-yl]oxy}phenyl)-N-[2-(4-fluorophenyl)acetyl]thiourea;
(6)N-(4-{[2-(1-methyl-1H-2-imidazolyl)thieno[3,2-b]pyridin-7-yl]o xy}phenyl)-N-(2-phenylacetyl)thiourea;
(7)N-(3-fluoro-4-{[2-(1-methyl-1H-2-imidazolyl)thieno[3,2-b]pyrid in-7-yl]oxy}phenyl)-N-(2-phenylacetyl)thiourea;
(8)N-(2-methoxy-4-{[2-(1-methyl-1H-2-imidazolyl)thieno[3,2-b]p yridin-7-yl]oxy}phenyl)-N-(2-phenylacetyl)thiourea;
(9)N-(2-methoxy-4-{[2-(1-methyl-1H-2-imidazolyl)thieno[3,2-b]p yridin-7-yl]oxy}phenyl)-N-(2-phenylacetyl)urea;
(10)N-[2-(1H-1-indazolyl)acetyl]-N-(4-{[2-(1-methyl-1H-2-imidaz olyl)thieno[3,2-b]pyridin-7-yl]oxy}phenyl)thiourea;
(11)N-(3-fluoro-4-{[2-(1-methyl-1H-2-imidazolyl)thieno[3,2-b]pyri din-7-yl]oxy}phenyl)-N-[2-(1H-1-indazolyl)acetyl]thiourea;
(12)N-(3-fluoro-4-{[2-(1-methyl-1H-2-imidazolyl)thieno[3,2-b]pyri din-7-yl]oxy}phenyl)-N-[2-(1H-1-indazolyl)acetyl]urea; and
(13)N-[2-(1H-1-indazolyl)acetyl]-N-(2-methoxy-4-{[2-(1-methyl-1 H-2-imidazolyl)thieno[3,2-b]pyridin-7-yl]oxy}phenyl)urea.

Examples of most preferred compounds in the first aspect of the present invention include the compound of Example 1 and the compound of Example 9.

### Compounds in second aspect of the invention

According to the second aspect of the present invention, there are provided quinoline derivatives and quinazoline derivatives.

R¹¹ and R¹² preferably represent methoxy.

D preferably represents CH.

G preferably represents O.

L is preferably absent or a halogen atom, preferably a fluorine or chlorine atom, at the 3-position, a halogen atom, preferably a fluorine atom, at the 2-position, or C₁₋₄ alkoxy, preferably methoxy, at the 2-position.

A combination of D, G, and M is preferably a combination in which D represents CH, G represents O, and M represents S, or a combination in which D represents CH, G represents O, and M represents O.

Q preferably represents formula (b): 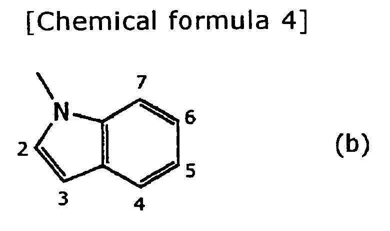 wherein the group represented by formula (b) is optionally substituted by a halogen atom and numerals represent substitutable positions;
formula (c): 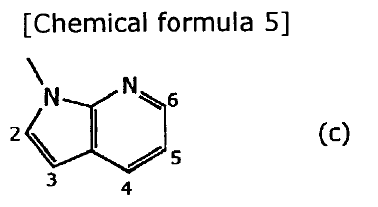 wherein the group represented by formula (c) is optionally substituted by a halogen atom and numerals represent substitutable positions;
formula (d): 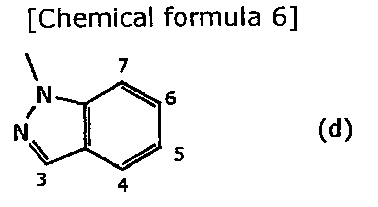 wherein the group represented by formula (d) is optionally substituted by a halogen atom and numerals represent substitutable positions; or
formula (e): 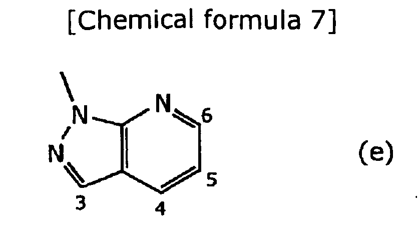 wherein the group represented by formula (e) is optionally substituted by a halogen atom and numerals represent substitutable positions.

Formulae (b), (c), (d), and (e) are preferably unsubstituted or have, as a substituent, a halogen atom, preferably a chlorine atom, at the 3-position; a halogen atom, preferably a fluorine atom, at the 4-position; or a halogen atom, preferably a fluorine atom, at the 7-position.

First examples of preferred compounds of formula (II) include compounds in which
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (b) wherein the group represented by formula (b) is optionally substituted by a halogen atom and the numerals represent substitutable positions.

In the first examples, L preferably represents a halogen atom, preferably a fluorine atom, at the 3-position.

In the first examples, M represents O.

Further, in the first examples, formula (b) is unsubstituted.

Second examples of preferred compounds of formula (II) include compounds in which
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (c) wherein the group represented by formula (c) is optionally substituted by a halogen atom and the numerals represent substitutable positions.

In the second examples, L preferably represents a halogen atom, preferably a fluorine atom, at the 3-position.

In the second examples, M preferably represents S.

In the second examples, formula (c) is unsubstituted.

Third examples of preferred compounds of formula (II) include compounds in which
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (d) wherein the group represented by formula (d) is optionally substituted by a halogen atom and the numerals represent substitutable positions.

In the third examples, L preferably a halogen atom, preferably a fluorine or chlorine atom, at the 3-position, C₁₋₄ alkoxy, preferably methoxy, at the 2-position, or a halogen atom, preferably a fluorine atom, at the 2-position.

In the third examples, M preferably represents O.

In the third examples, M preferably represents S.

In the third examples, formula (d) is preferably unsubstituted or have, as a substituent, a halogen atom, preferably a chlorine atom, at the 3-position: a halogen atom, preferably a fluorine atom, at the 4-position; or a halogen atom, preferably a fluorine atom, at the 7-position.

Fourth examples of preferred compounds of formula (II) include compounds in which
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (e) wherein the group represented by formula (e) is optionally substituted by a halogen atom and the numerals represent substitutable positions.

In the fourth examples, L preferably represents a halogen atom, preferably a fluorine atom, at the 3-position, or C₁₋₄ alkoxy, preferably methoxy, at the 2-position.

In the fourth examples, M preferably represents O.

In the fourth examples, M preferably represents S.

In the fourth examples, formula (e) is preferably unsubsituted.

Preferred compounds among the compounds of formula(II) are as follows. The number within the parentheses correspond to Example No.

(14)N-[4-{(6,7-dimethoxy-4-quinolyl)oxy}-3-fluorophenyl]-N'-[2-( 1H-1-indazolyl)acetyl]urea;
(15)N-[4-{(6,7-dimethoxy-4-quinolyl)oxy}-2-methoxyphenyl]-N'-[ 2-(1H-1-indazolyl)acetyl]thiourea;
(16)N-[4-{(6,7-dimethoxy-4-quinolyl)oxy}-3-fluorophenyl]-N'-[2-( 1H-1-indolyl)acetyl]urea;
(17)N-[4-{(6,7-dimethoxy-4-quinolyl)oxy}-2-methoxyphenyl]-N'-[ 2-(1H-1-indazolyl)acetyl]urea;
(18)N-[4-{(6,7-dimethoxy-4-quinolyl)oxy}-3-fluorophenyl]-N'-[2-( 1H-1-indazolyl)acetyl]thiourea;
(19)N-[4-{(6,7-dimethoxy-4-quinolyl)oxy}-2-fluorophenyl]-N'-[2-( 1H-1-indazolyl)acetyl]urea;
(20)N-[2-(3-chloro-1H-1-indazolyl)acetyl]-N'-[4-{(6,7-dimethoxy-4-quinolyl)oxy}phenyl]urea;
(21)N-[2-(3-chloro-1H-1-indazolyl)acetyl]-N'-[4-{(6,7-dimethoxy-4-quinolyl)oxy}-3-fluorophenyl]urea;
(22)N-[3-chloro-4-{(6,7-dimethoxy-4-quinolyl)oxy}phenyl]-N'-[2-( 1H-1-indazolyl)acetyl]urea;
(23)N-[4-{(6,7-dimethoxy-4-quinolyl)oxy}-3-fluorophenyl]-N'-[2-( 1H-pyrrolo[2,3-b]pyridin-1-yl)acetyl]thiourea;
(24)N-[4-[{6,7-dimethoxy-4-quinolyl}oxy]-2-fluorophenyl]-N'-[2-( 4-fluoro-1H-1-indazolyl)acetyl]thiourea;
(25)N-[4-{(6,7-dimethoxy-4-quinolyl)oxy}-3-fluorophenyl]-N'-[2-( 4-fluoro-1H-1-indazolyl)acetyl]thiourea;
(26)N-[4-{(6,7-dimethoxy-4-quinolyl)oxy}-2-methoxyphenyl]-N'-[ 2-(4-fluoro-1H-1-indazolyl)acetyl]thiourea;
(27)N-[4-{(6,7-dimethoxy-4-quinolyl)oxy}phenyl]-N'-[2-(4-fluoro-1H-1-indazolyl)acetyl]urea;
(28)N-[4-{(6,7-dimethoxy-4-quinolyl)oxy}-2-fluorophenyl]-N'-[2-( 4-fluoro-1H-1-indazolyl)acetyl]thiourea;
(29)N-[4-{(6,7-dimethoxy-4-quinolyl)oxy}-2-methoxyphenyl]-N'-[ 2-(4-fluoro-1H-1-indazolyl)acetyl]urea;
(30)N-[4-{(6,7-dimethoxy-4-quinolyl)oxy}-3-fluorophenyl]-N'-[2-( 4-fluoro-1H-1-indazolyl)acetyl]urea;
(31)N-[4-{(6,7-dimethoxy-4-quinolyl)oxy}-3-fluorophenyl]-N'-[2-( 1H-pyrazolo[3,4-b]pyridin-1-yl)acetyl]thiourea;
(32)N-[4-{(6,7-dimethoxy-4-quinolyl)oxy}-2-methoxyphenyl]-N'-[ 2-(7-fluoro-1H-1-indazolyl)acetyl]thiourea;
(33)N-[4-{(6,7-dimethoxy-4-quinolyl)oxy}-2-methoxyphenyl]-N'-[ 2-(7-fluoro-1H-1-indazolyl)acetyl]urea;
(34)N-[4-{(6,7-dimethoxy-4-quinolyl)oxy}-2-methoxyphenyl]-N'-[ 2-(1H-1-pyrazolo[3,4-b]pyridin-1-yl)acetyl]urea; and
(35)N-[4-{(6,7-dimethoxy-4-quinolyl)oxy}-3-fluorophenyl]-N'-[2-( 1H-1-pyrazolo[3,4-b]pyridin-1-yl)acetyl]urea.

According to the second aspect of the present invention, there are also provided the following compounds.

(A)N-[4-{(6,7-dimethoxy-4-quinolyl)oxy}-2-methoxyphenyl]-N'-[2 -(4-fluorophenyl)acetyl]thiourea; and
(B)N-{2-methoxy-4-[6-methoxy-7-(3-morpholinopropoxy)-4-quino lyl]oxyphenyl}-N'-(2-phenylacetyl)thiourea.

The compound of Example 14 and compound A may be mentioned as the most preferred compounds in the second aspect of the present invention.

The compounds in the first aspect of the present invention, the compounds in the second aspect of the present invention, and their salts may form pharmaceutically acceptable salts thereof. Preferred examples of such salts include: alkali metal or alkaline earth metal salts such as sodium salts, potassium salts or calcium salts; hydrohalogenic acid salts such as hydrofluoride salts, hydrochloride salts, hydrobromide salts, or hydroiodide salts; inorganic acid salts such as nitric acid salts, perchloric acid salts, sulfuric acid salts, or phosphoric acid salts; alkylsulfonic acid salts such as methanesulfonic acid salts, trifluoromethanesulfonic acid salts, or ethanesulfonic acid salts; arylsulfonic acid salts such as p-toluenesulfonic acid salts; organic acid salts such as fumaric acid salts, succinic acid salts, citric acid salts, tartaric acid salts, oxalic acid salts, maleic acid salts, acetic acid salts, malic acid salts, lactic acid salts, or ascorbic acid salts; and amino acid salts such as glycine salts, phenylalanine salts, glutamic acid salts, or aspartic acid salts.

The compounds in the first aspect of the present invention, the compounds in the second aspect of the present invention, and their salts may form solvates. Such solvates include, for example, hydrates, alcoholates, for example, methanolates and ethanolates, and etherates, for example, diethyl etherate.

KDR inhibitors having a KDR autophosphorylation inhibitory activity has been confirmed to be useful for various cancers including gastric cancers but at the same time has been reported to induce side effects such as blood pressure elevation and disorder of the kidney (Bull. Cancer, 92 S29 (2005)). Further, there is also a report that the KDR inhibitor has significant activity to induce thickening of a bone growth plate of animals at the growth stage (Cancer Res. 65, 4389 (2005)). This fact suggests that the same phenomenon may be induced upon administration to a patient at the growth stage. For the above reason, when the treatment of human is contemplated, it is preferable that the compound having c-Met autophosphorylation inhibitory activity should have low KDR autophosphorylation inhibitory activity, that is, has high c-Met/KDR selectivity from the viewpoint of reducing the above side effect.

A part of compounds according to the present invention have high c-Met autophosphorylation inhibitory activity, but on the other hand, have low KDR autophosphorylation inhibitory activity. Such compounds are considered to have a low possibility of inducing side effect attributable to KDR autophosphorylation inhibitory action. Examples of compounds, which have high c-Met autophosphorylation inhibitory activity, but on the other hand, have low KDR autophosphorylation inhibitory activity, include compounds A and B. In an actual experiment, compound A did not induce the thickening of a bone growth plate of animals at the growth stage (Pharmacological Test Example 9). On the other hand, in a nude mouse tumor model in which a human gastric cancer cell line, which expressed c-Met protein on a high level, has been subcutaneously implanted, compound A was confirmed to have antitumor activity upon the administration of compound A by a method similar to the above bone growth plate thickening induction test (Pharmacological Test Example 5).
When the treatment of human is contemplated, it is preferable that the compound having a c-Met autophosphorylation inhibitory activity should have a proper metabolic rate which is preferable as a therapeutic agent. In a human liver microsome test, a part of the compounds according to the present invention have a proper metabolic rate which is preferable as a therapeutic agent. Compound A can be mentioned as an example of such compounds (data not shown).

### Production of compounds

Compounds according to the present invention may be produced, for example, according to schemes 1 to 7 below. Starting compounds necessary for the synthesis of the compounds according to the present invention are commercially available or alternatively can be easily produced by conventional methods. Substituents in the scheme are as defined in formula (I), formula (II), and formula (a).

<Production of 4-(aminophenoxy)thienopyridine derivatives (scheme 1)>

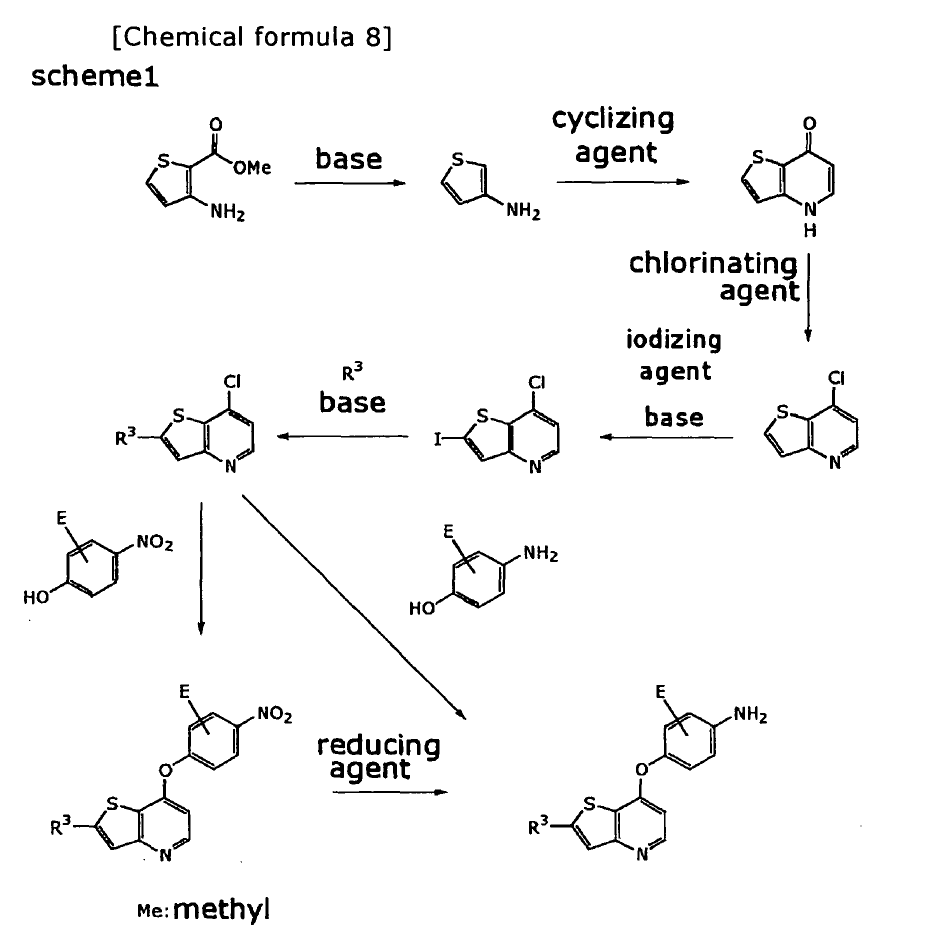

7-Chlorothienopyridine derivatives may be synthesized by conventional methods as described, for example, in Org. Synth. Col. Vol. 3, 272 (1955), Acta Chim. Hung., 112, 241(1983), WO98/47873, or WO 99/24440. Synthesis examples of 7-chlorothienopyridine derivatives are as shown in sheme 1.

3-Aminothiophene may be produced by first allowing a 3-aminothiophene derivative to react in a suitable basic solution, for example, an aqueous sodium hydroxide solution, under reflux and then subjecting the reaction product to acid treatment. Thienopyridone may be produced by allowing 3-aminothiophene to react in a suitable solvent, for example, triethyl o-formate, in the presence of a suitable cyclizing agent, for example, 2,2-dimethyl-1,3-dioxane-4,6-dione, and then heating the reaction product in a suitable solvent, for example, a mixed solution composed of diphenyl ether and ether). 7-Chlorothienopyridine may be synthesized by allowing thienopyridone to react in the presence of a chlorinating agent, for example, phosphorus oxychloride. 2-Iodo-7-chlorothienopyridine may be produced by allowing 7-chlorothienopyridine to react in a suitable solvent, for example, tetrahydrofuran, in the presence of a base, for example, n-butyllithium and an iodizing agent, for example, iodine. A 7-chlorothienopyridine derivative may be produced by activating a reagent corresponding to R³ with a suitable base, for example, n-butyllithium, in a suitable solvent, for example, tetrahydrofuran, subjecting the activated reagent to metal replacement with a suitable metal salt, for example, dichlorozinc, and then allowing the product to react with 2-iodo-7-chlorothienopyridine in the presence of a suitable catalyst, for example, tetrakis(triphenylphosphine)palladium.

Next, a 7-(aminophenoxy)thienopyridine derivative is produced by reacting a nitrophenol derivative with the 7-chlorothienopyridine derivative in a suitable solvent, for example, chlorobenzene, to synthesize a 7-(nitrophenoxy)thienopyridine derivative and then reacting the 7-(nitrophenoxy)thienopyridine derivative in a suitable solvent, for example, N,N-dimethyl formamide, in the presence of a catalyst, for example, palladium hydroxide-carbon, palladium-carbon, under a hydrogen atmosphere. The nitro group can also be reduced with zinc, iron or the like.

Alternatively, the 7-(aminophenoxy)thienopyridine derivative may be produced by reacting an aminophenol derivative with the 7-chlorothienopyridine derivative in a suitable solvent, for example, dimethyl sulfoxide, in the presence of a base, for example, sodium hydride.

A 7-chlorothienopyrimidine derivative may also be synthesized from the same starting material as in scheme 1 by a method similar to scheme 3.

<Production of compound of formula (I) (scheme 2)>

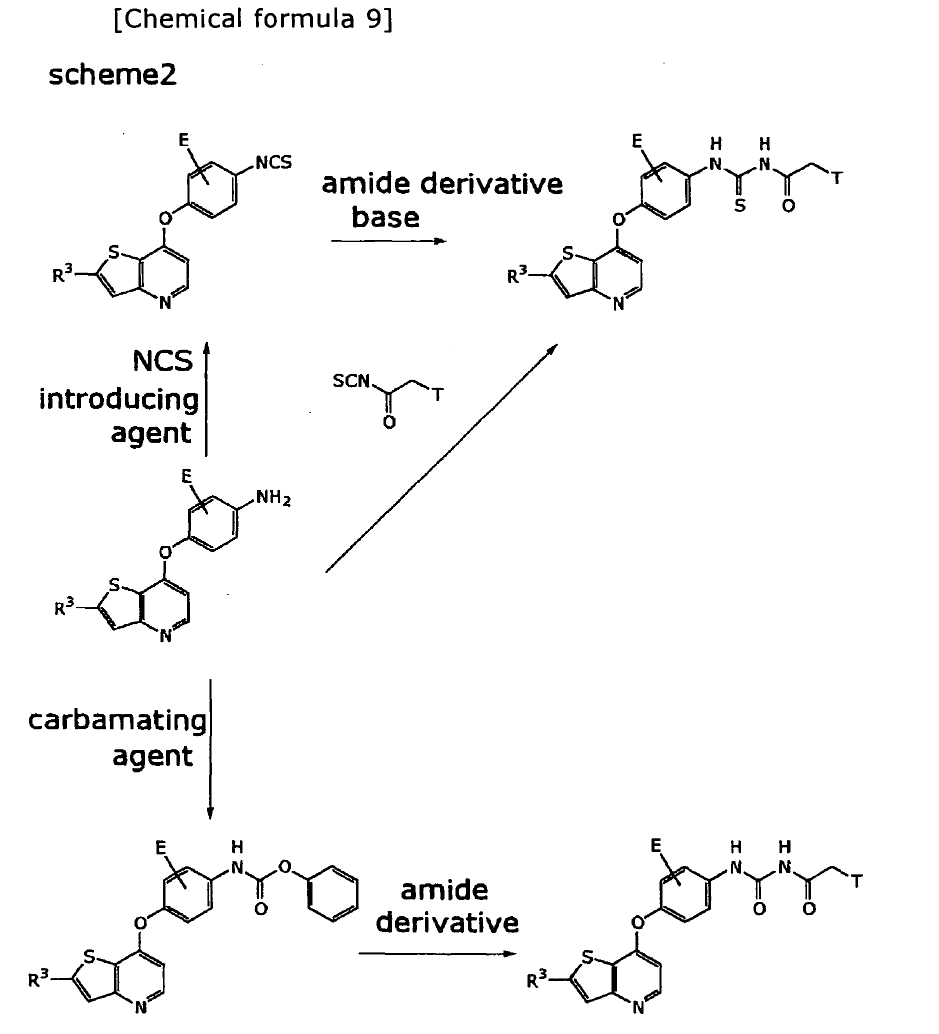

Compounds of formula (I) may be produced according to scheme 2. A carbonylthiourea derivative may be produced by reacting a 4-(aminophenoxy)thienopyridine derivative with a carbonyl thioisocyanate derivative in a suitable solvent, for example, a mixed solvent composed of toluene and ethanol. The carbonyl thioisocyanate derivative is commercially available or can be easily produced by a conventional method. For example, the carbonyl thioisocyanate derivative is produced by reacting an acid chloride derivative with potassium thiocyanate in a suitable solvent, for example, acetonitrile.

Alternatively, a thioisocyanate derivative may be produced by reacting a 4-(aminophenoxy)thienopyridine derivative with a thioisocyanating agent, for example, di-2-pyridyl thiocarbonate, in a suitable solvent, for example, chloroform. A carbonylthiourea derivative may be produced by reacting the thioisocyanate derivative thus obtained with an amide derivative in a suitable solvent, for example, dimethylformamide, in the presence of a suitable base, for example, sodium hydride.

A carbamate derivative may be produced by reacting a 4-(aminophenoxy)thienopyridine derivative with a carbamating agent, for example, phenyl chloroformate, in a suitable solvent, for example, dimethylformamide, in the presence of a suitable base, for example, sodium hydride.

A carbonylurea derivative may be produced by reacting the carbamate derivative thus obtained with an amide derivative in a suitable solvent, for example, o-xylene.

<Production of 4-chloroquinoline derivative and 4-chloroquinazoline derivative (scheme 3)>

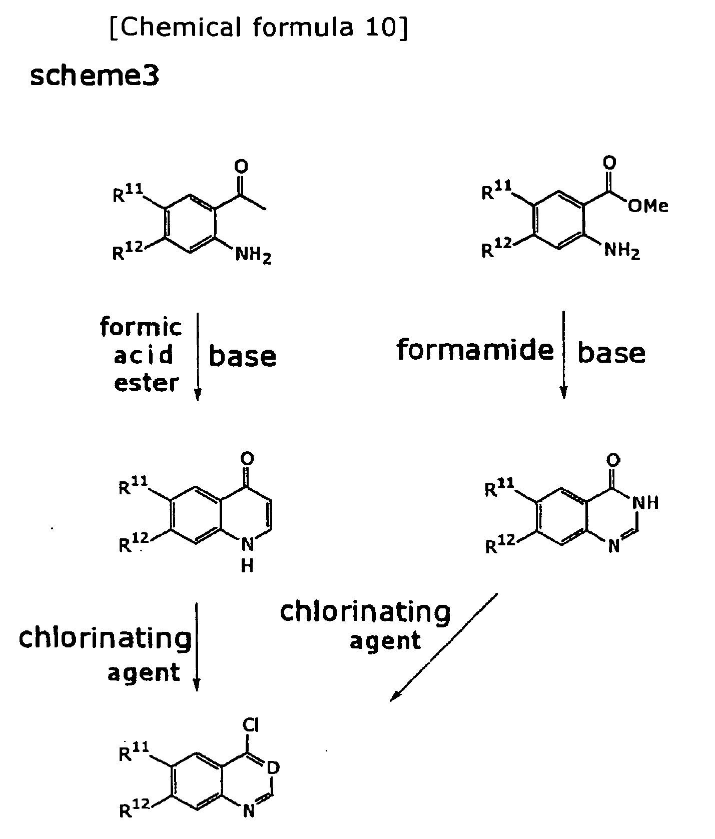

The 4-chloroquinoline derivative may be synthesized by a conventional method described, for example, in Org. Synth. Col. Vol. 3, 272 (1955), Acta Chim. Hung.,112, 241 (1983), or WO98/47873. An example of synthesis of 4-chloroquinoline derivatives is as shown in scheme 3.

A quinolone derivative may be produced by reacting a 2-amioacetophenone derivative with a formic acid ester, for example, ethyl formate, in a suitable solvent, for example, tetrahydrofuran, in the presence of a base, for example, sodium methoxide. A 4-chloroquinoline derivative may be produced by allowing the quinolone derivative to react in the presence of a chlorinating agent, for example, phosphorus oxychloride.

4-Chloroquinazoline derivatives may be produced, for example, as follows. A quinazolone derivative may be produced by reacting a 2-aminobenzoic acid ester derivative with formamide in a suitable solvent, for example, a mixed solvent composed of N,N-dimethylformamide and methanol, in the presence of a base, for example, sodium methoxide. A 4-chloroquinazoline derivative may be produced by allowing the quinazolone derivative to react in the presence of a chlorinating agent, for example, phosphorus oxychloride.

<Production of aniline derivatives having quinoline ring or quinazoline ring (G=O) (scheme 4)>

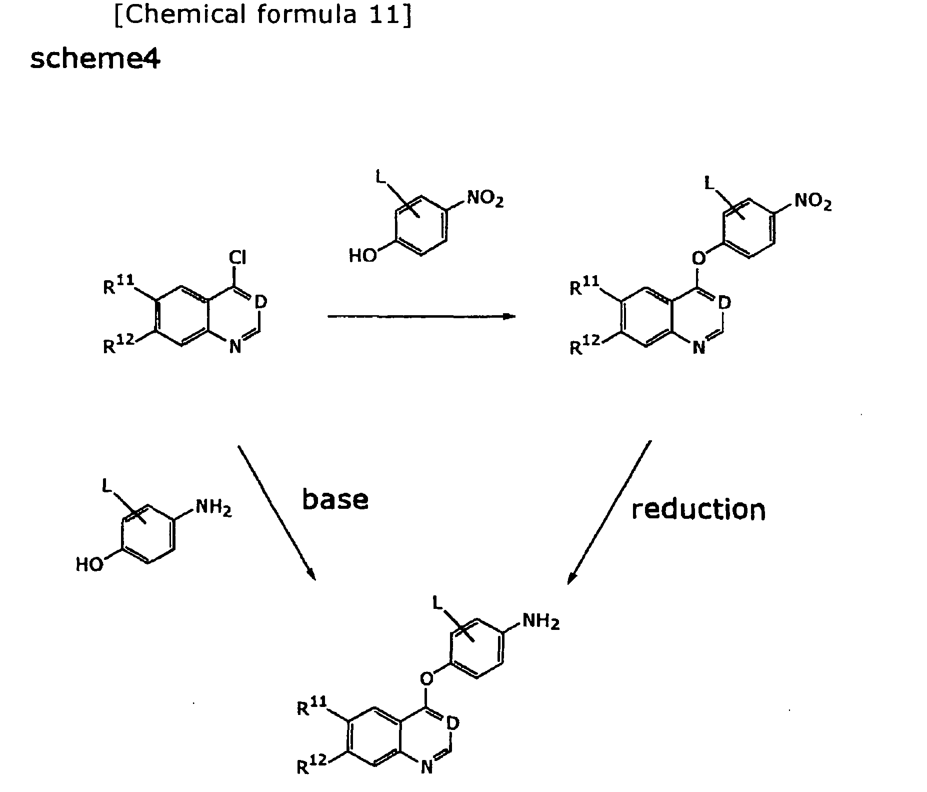

Aniline derivatives having quinoline ring or quinazoline ring (G=O) may be produced, for example, according to scheme 4.

Specifically, a 4-(aminophenoxy)quinoline derivative or a corresponding quinazoline derivative may be produced by reacting a nitrophenol derivative with a 4-chloroquinoline derivative or a corresponding quinazoline derivative in a suitable solvent, for example, chlorobenzene, to give a 4-(nitrophenoxy)quinoline derivative or a corresponding quinazoline derivative and then allowing the product to react in a suitable solvent, for example, N,N-dimethylformamide, in the presence of a catalyst, for example, palladium hydroxide-carbon or palladium-carbon, under a hydrogen atmosphere. The nitro group may also be reduced, for example, with zinc or iron.

Alternatively, a 4-(aminophenoxy)quinoline derivative or a corresponding quinazoline derivative may be produced by reacting an aminophenol derivative with a 4-chloroquinoline derivative or a corresponding quinazoline derivative in a suitable solvent, for example, dimethylsulfoxide, in the presence of a base, for example, sodium hydride. The 4-(aminophenoxy)quinazoline derivative may also be produced by dissolving an aminophenol derivative in an aqueous sodium hydroxide solution and subjecting the solution to a two-phase reaction with a 4-chloroquinazoline derivative dissolved in a suitable organic solvent, for example, ethyl methyl ketone, in the presence of a phase transfer catalyst, for example, tetra-n-butylammonium chloride or in the absence of a catalyst.

<Production of aniline derivatives having quinoline ring or quinazoline ring (G=S) (scheme 5)>

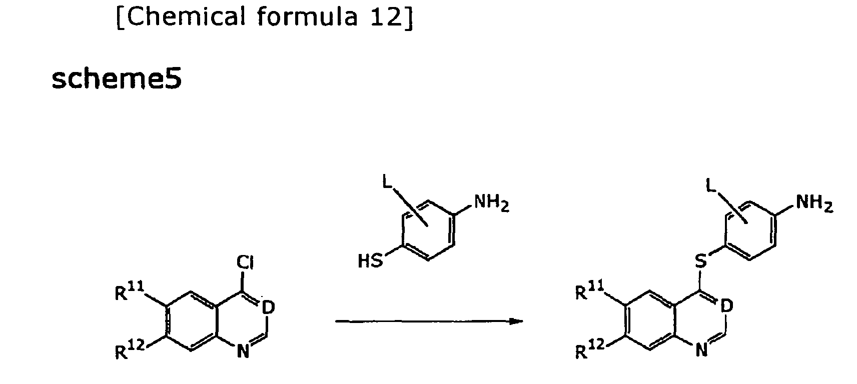

Aniline derivatives having quinoline ring or quinazoline ring (G=S) may be produced, for example, according to scheme 5.

Specifically, a 4-(quinolylsulfanyl)aniline derivative or a 4-(quinazolinylsulfanyl)aniline derivative (a compound of formula (II) wherein G = S) may be produced by reacting an aminothiophenol derivative with a 4-chloroquinoline derivative or a corresponding quinazoline derivative in a suitable solvent, for example, chlorobenzene. A derivative having a sulfur atom at the 4-position of a quinoline or quinazoline ring may be produced from this derivative according to scheme 7.

<Synthesis of reagent corresponding to Q in formula (II) (scheme 6)>

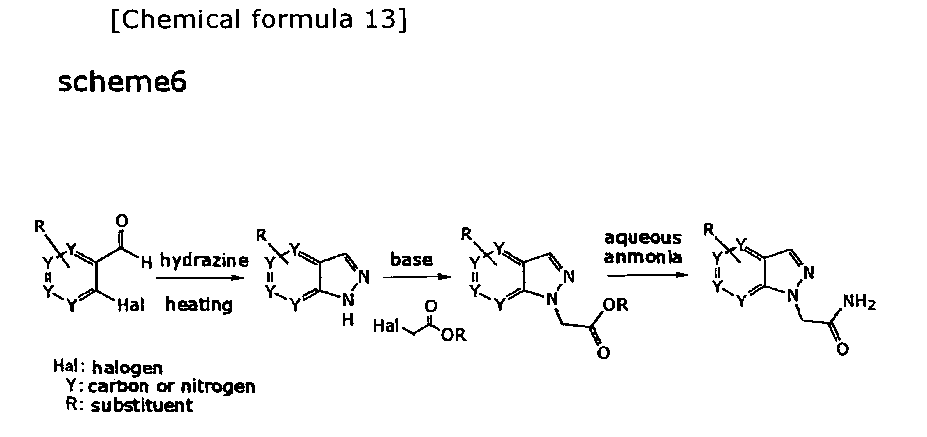

The material for a heterocondensed ring corresponding to Q is commercially available or alternatively may be synthesized by a conventional method. As shown in scheme 6, indazole derivatives and the like may be synthesized as follows. Specifically, a corresponding indazole derivative may be produced by heating benzaldehyde substituted at its o-position by a halogen or the like and hydrazines. Further, the corresponding indazole derivative may be converted to an amide compound as an important intermediate compound by reacting the indazole derivative with an α-haloacetic ester compound, for example, methyl bromoacetate, in the presence of a suitable base, for example, sodium hydride, to give an acetic ester derivative and then treating the acetic ester derivative with aqueous ammonia.

<Production of compound of formula (II) (scheme 7)>

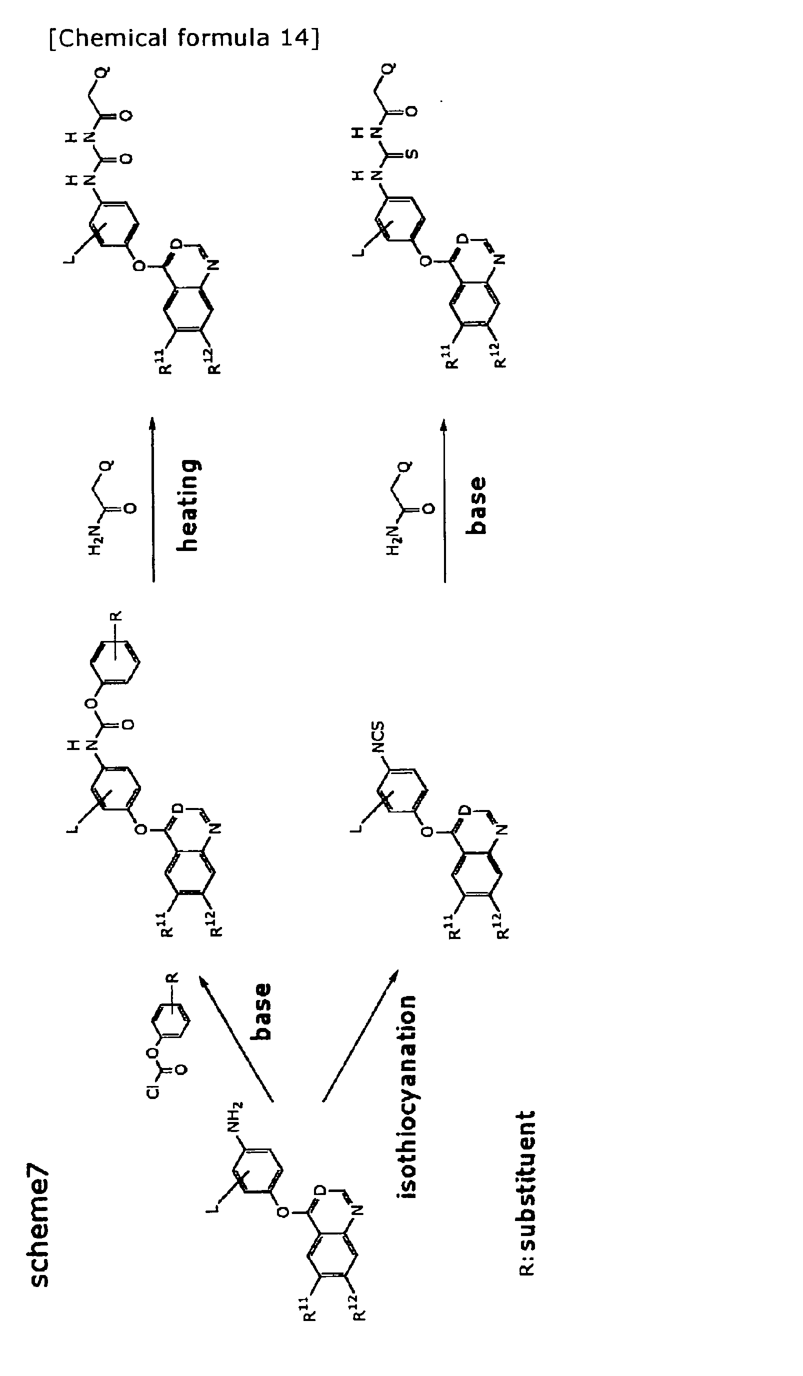

The compound of formula (II), that is, a derivative having carbonylurea or carbonylthiourea may be synthesized according to scheme 7.

The compound of formula (II) having carbonylurea (M = O) may be produced by leading the aniline compound to a phenyl carbamate compound, then adding an amide as the material, and heating the mixture.

A compound of formula (II) having carbonylthiourea (M = S) may be produced by leading an aniline compound with di-2-pyridyl thiocarbamate or the like to an isothiocyanate compound and then adding an amide as the material in the presence of a base, for example, sodium hydride.

### Use of compounds

The compounds according to the present invention have tumor growth inhibitory activity in vivo (see Pharmacological Test Examples 4, 5, and 6).

Further, the compounds according to the present invention inhibit in vitro the c-Met autophosphorylation caused by the stimulation of human epidermoid cancer cells A431 with HGF and the c-Met autophosphorylation which constantly occurs in gastric cancer cells MKN45 non-dependently upon HGF (see Pharmacological Test Examples 2 and 3).

Upon HGF stimulation or in a HGF-non-dependent manner for certain cancer cells, c-Met accelerates proliferation and motility in various cell species through the autophosphorylation of intracellular region with tyrosine kinase (J. Biochem., 119, 591, (1996), Jpn. J. Cancer Res., 88, 564, (1997), and Int. J. Cancer, 78, 750, (1998)). In particular, in a plurality of cancers, for example, the enhancement of HGF concentration in the blood, excessive expression of c-Met, and the expression of c-Met mutants which have acquired HGF non-dependency are reported. c-Met signals are considered to be involved in the proliferation, invasion and metastasis of various cancer cells (Int. J. Cancer, 55, 72, (1993), Oncology Reports, 5, 1013 (1998), Proc. Natl. Acad. Sci. USA, 88, 4892, (1991), and Cancer, 88, 1801, (2000)). Further, it is also reported that HGF accelerates through c-Met the proliferation and migration activity of vascular endothelial cells and accelerates angiogenesis (Circulation, 97, 381 (1998) and Clinical Cancer Res., 5, 3695, (1999)), and, consequently, it is estimated that HGF is also related to angiogenesis in cancers.

Accordingly, the compounds according to the present invention can inhibit the growth, invasion, metastasis, and angiogenesis of cancer cells and thus can be used in the therapy of malignant tumors.

According to the present invention, there is provided a pharmaceutical composition comprising the compound in the first aspect of the present invention or the compound in the second aspect of the present invention.

The pharmaceutical composition according to the present invention can be used in the treatmant of malignant tumors such as cerebral tumors, gastric cancer, colon cancer, pancreatic cancer, lung cancer, renal cancer, ovarian cancer, and prostatic cancer.

Further, according to the present invention, there is provided a method for treating a malignant tumor, comprising the step of administering a therapeutically effective amount of the compound in the first aspect of the present invention or the compound in the second aspect of the present invention together with a pharmaceutically acceptable carrier to a mammal including a human to be treated.

Furthermore, according to the present invention, there is provided use of the compound in the first aspect of the present invention or the compound in the second aspect of the present invention, for the manufacture of a therapeutic agent for a malignant tumor.

c-Met autophosphorylation inhibitors can be used in studying the role of HGF/c-Met signals or downstream signal transduction, for example, in various cancer cells, vascular endothelial cells, epithelial cells, blood cells, and hepatic cells by blocking of HGF/c-Met signals. Antisense techniques and siRNA techniques have recently been applied to such studies. However, these are techniques for controlling protein expression. The influence of kinase activity inhibition can be examined only by a kinase inhibitor. Therefore, the compound according to the present invention is useful as a reagent for studying the influence of c-Met autophosphorylation inhibition in vivo.

Accordingly, the present invention provides a c-Met autophosphorylation inhibitor comprising a compound according to the present invention.

Further, according to the present invention, there is provided an agent for researching HGF/c-Met signal transduction.

### Pharmaceutical composition

The compounds according to the present invention can be administered to human and non-human animals orally or parenterally by administration routes, for example, intravenous administration, intramuscular administration, subcutaneous administration, rectal administration, or percutaneous administration. Therefore, the pharmaceutical composition comprising as an active ingredient the compound according to the present invention is formulated into suitable dosage forms according to the administration routes. Specifically, oral preparations include tablets, capsules, powders, granules, and syrups, and parental preparations include injections, suppositories, tapes, and ointments.

These various preparations may be prepared by conventional methods, for example, with commonly used excipients, disintegrants, binders, lubricants, colorants, and diluents.

Excipients include, for example, lactose, glucose, corn starch, sorbit, and crystalline cellulose. Disintegrants include, for example, starch, sodium alginate, gelatin powder, calcium carbonate, calcium citrate, and dextrin. Binders include, for example, dimethylcellulose, polyvinyl alcohol, polyvinyl ether, methylcellulose, ethylcellulose, gum arabic, gelatin, hydroxypropylcellulose, and polyvinyl pyrrolidone. Lubricants include, for example, talc, magnesium stearate, polyethylene glycol, and hydrogenated vegetable oils.

In preparing the injections, if necessary, for example, buffers, pH adjustors, stabilizers, tonicity agents, and preservatives may be added.

The content of the compound according to the present invention in the pharmaceutical composition according to the present invention may vary depending upon the dosage form. In general, however, the content is 0.5 to 50% by weight, preferably 1 to 20% by weight, based on the whole composition.

The dose may be appropriately determined in consideration of, for example, the age, weight, sex, difference in diseases, and severity of condition of individual patients, preferably in the range of 1 to 100 mg/kg. This dose is administered at a time daily or divided doses of several times daily.

The compound according to the present invention may be administered in combination with other medicament(s), for example, a carcinostatic agent. In this case, the compound according to the present invention may be administered simultaneously with or after or before the administration of other medicament(s). The type, administration intervals and the like of the carcinostatic agent may be determined depending upon the type of cancer and the condition of patients.

### EXAMPLES

The present invention is further illustrated by the following Examples that are not intended as a limitation of the invention.

Materials necessary for synthesis were produced by methods described in WO 97/17329, WO 98/47873, WO 00/43366, Japanese Patent Laid-Open No. 328782/1997, WO 03/000660, and WO 04/039782. Regarding materials not described in these publications, production methods for these materials are described as Production Examples.

### Example 1:

N-(3-Chloro-4-{[2-(1-methyl-1H-2-imidazolyl)thieno[3,2-b]pyri din-7-yl]oxy}phenyl)-N-[2-(4-fluorophenyl)-acetyl]thiourea

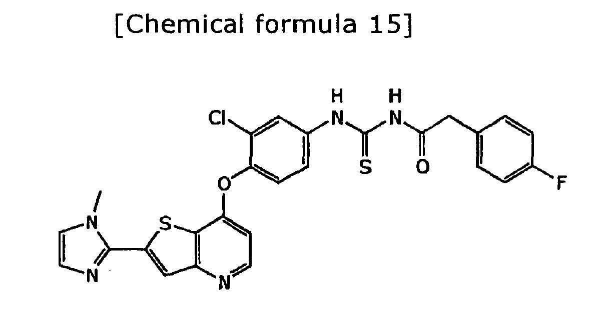

4-Fluorophenylacetylchloride [starting material B] (124 mg) and potassium thiocyanate (87 mg) were dissolved in acetonitrile (3 ml), and the mixture was stirred at 50°C for one hr. Acetonitrile was removed by evaporation under the reduced pressure. A saturated aqueous sodium hydrogencarbonate solution and ethyl acetate were then added to the residue, and the mixture was extracted with ethyl acetate. Ethyl acetate was removed by evaporation under the reduced pressure, and the crude product thus obtained was dissolved in toluene/ethanol (1/1). 3-Chloro-4-{[2-(1-methyl-1H-2-imidazolyl)thieno[3,2-b]pyridin-7-yl]oxy}aniline [starting material A] (65 mg) was added to the solution, and the mixture was stirred at room temperature for one hr. Water was added to the reaction solution, and the mixture was extracted with chloroform, and chloroform was removed by evaporation under the reduced pressure. The residue was purified by chromatography on silica gel developed with chloroform/acetone to give the title compound (70 mg, yield 71%).

¹H-NMR (CDCl₃, 400 MHz): δ 3.72 (s, 2H), 3.97 (s, 3H), 6.61 (d, J = 5.6 Hz, 1H), 7.03 (d, J = 1.0 Hz, 2H), 7.13 (t, J = 8.5 Hz, 2H), 7.17 (d, J = 1.2 Hz, 1H), 7.27 - 7.31 (m, 2H), 7.62 (dd, J = 2.7 Hz, 8.8 Hz, 1H), 7.72 (s, 1H), 8.00 (d, J = 2.7 Hz, 1H), 8.50 (d, J = 5.4 Hz, 1H), 8.52 (s, 1H), 12.39 (s, 1H)
Mass spectrometric value (m/z): 552 [M + H]⁺

### Example 2:

N-(3-Fluoro-4-{[2-(1-methyl-1H-2-imidazolyl)thieno[3,2-b]pyri din-7-yl]oxy}phenyl)-N-(2-phenylacetyl)urea

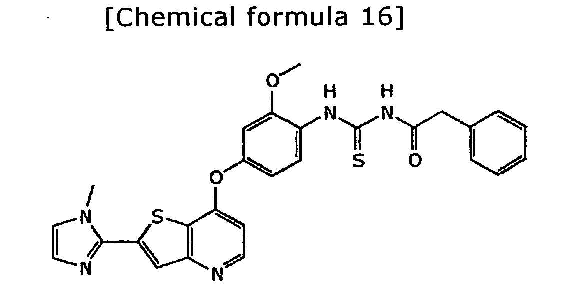

3-Fluoro-4-{[2-(1-methyl-1H-2-imidazolyl)thieno[3,2-b]p yridin-7-yl]oxy}aniline [starting material A] (130 mg) was dissolved in dimethylformamide (5 ml). Sodium hydride (23 mg) was added to the solution, and the mixture was stirred at room temperature for 30 min. Phenyl chloroformate (73 µl) was added thereto, and the mixture was stirred at room temperature for one hr. Further, ammonium chloride (5 mg) was added thereto, and the mixture was stirred for 10 min. Water was added to the reaction solution, the mixture was extracted with ethyl acetate. Ethyl acetate was removed by evaporation under the reduced pressure. The residue was purified by chromatography on silica gel developed with chloroform/ethyl acetate to give phenyl N-(3-fluoro-4-{[2-(1-methyl-1H-2-imidazolyl)thieno[3,2-b]pyrid in-7-yl]oxy}phenyl) carbamate (72 mg, yield 41%). The Phenyl N-(3-fluoro-4-{[2-(1-methyl-1H-2-imidazolyl)thieno[3,2-b]pyrid in-7-yl]oxy}phenyl) carbamate (35 mg) and 2-phenylacetamide [starting material B] (12 mg) were dissolved in o-xylene (5 ml), and the solution was stirred at 185°C for 4 hr. The o-xylene was removed by evaporation under the reduced pressure and was then purified by chromatography on silica gel developed with chloroform/methanol to give the title compound (8 mg, yield 22%).

¹H-NMR (CDCl₃, 400 MHz): δ 3.77 (s, 2H), 3.97 (s, 3H), 6.54 (dd, J = 1.0 Hz, 5.4 Hz, 1H), 7.03 (d, J = 1.0 Hz, 1H), 7.17 (d, J = 1.0 Hz, 1H), 7.19 - 7.45 (m, 8H), 7.70 (s, 1H), 7.83 (s, 1H), 8.49 (d, J = 5.4 Hz, 1H), 10.64 (s, 1H)
Mass spectrometric value (m/z): 500 [M - H]⁻

### Example 3:

N-[2-(1H-1-Indazoly)acetyl]-N-(2-methoxy-4-{[2-(1-methyl-1H -2-imidazolyl)thieno[3,2-b]pyridin-7-yl]oxy}phenyl)thiourea

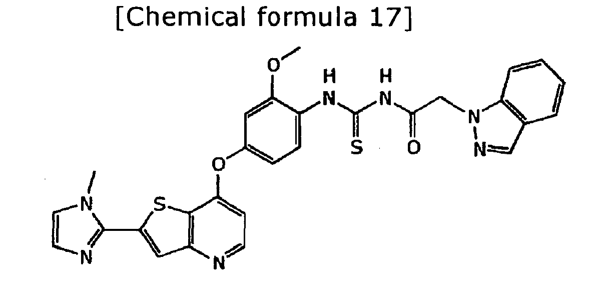

2-Methoxy-4-{[2-(1-methyl-1H-2-imidazolyl)thieno[3,2-b ]pyridin-7-yl]oxy}aniline [starting material A] (24 mg) and di-2-pyridyl thiocarbonate (19 mg) were dissolved in chloroform (5 ml), and the solution was stirred at room temperature for one hr. Water was added to the reaction solution, and the mixture was extracted with chloroform. Chloroform was removed by evaporation under the reduced pressure, and the residue was purified by chromatography on silica gel developed with chloroform/methanol to give 2-methoxy-4-{[2-(1-methyl-1H-2-imidazolyl)thieno[3,2-b]pyridi n-7-yl]oxy}phenylisothiocyanate. 2-(1H-1-Indazolyl)acetamide [starting material B] (11 mg) and sodium hydride (2.8 mg) were dissolved in dimethylformamide (2 ml), and the mixture was stirred at room temperature for 20 min. A solution of 2-methoxy-4-{[2-(1-methyl-1H-2-imidazolyl)thieno[3,2-b]pyridi n-7-yl]oxy}phenylisothiocyanate dissolved in dimethylformamide (4 ml) was added thereto, and the mixture was stirred at room temperature for 2 hr. Water was added to the reaction solution, and the mixture was extracted with ethyl acetate. Ethyl acetate was removed by evaporation under the reduced pressure, and the residue was purified by chromatography on silica gel developed with chloroform/methanol to give the title compound (11 mg, yield 36%).

¹H-NMR (CDCl₃, 400 MHz): δ 3.91 (s, 3H), 3.97 (s, 3H), 5.20 (s, 2H), 6.66 (d, J = 5.6 Hz, 1H), 6.79 - 6.81 (m, 2H), 7.04 (s, 1H), 7.17 (s, 1H), 7.26 - 7.29 (m, 1H), 7.44 (d, J = 8.5 Hz, 1H), 7.49 - 7.51 (m, 1H), 7.72 (s, 1H), 7.83 (d, J = 8.3 Hz, 1H), 8.25 (s, 1H), 8.50 (d, J = 5.6 Hz, 1H), 8.75 (d, J = 9.5 Hz, 1H), 9.22 (s, 1H), 12.39 (s, 1H)
Mass spectrometric value (m/z): 568 [M - H]⁻
Compounds of Examples 4 to 13 were synthesized according to the methods described in Examples 1, 2 and 3. The chemical structural formulae, starting materials, and synthesis methods respectively for the compounds are shown in Table 1.

**[Table 1]**

| Example No. | Structure of compound | Starting material A | Starting material B | Synthesis method |
|---|---|---|---|---|
| 4 | 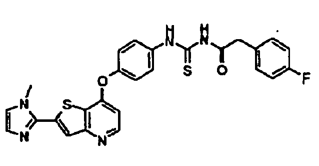 | 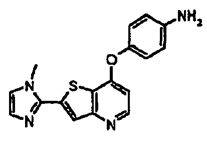 | 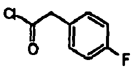 | Ex. 1 |
| 5 | 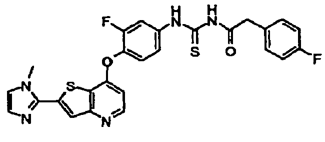 | 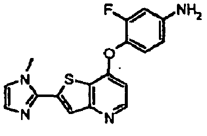 | 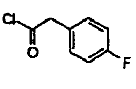 | Ex. 1 |
| 6 | 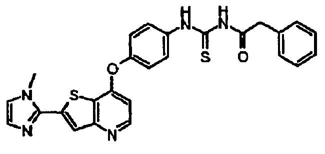 | 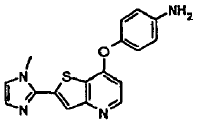 | 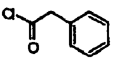 | Ex. 1 |
| 7 | 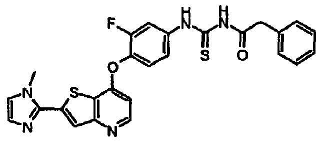 | 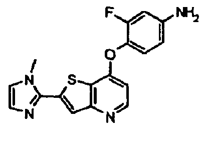 | 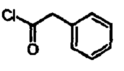 | Ex. 1 |
| 8 | 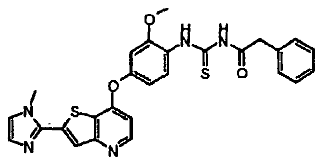 | 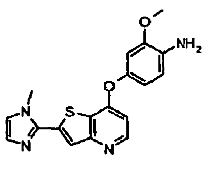 | 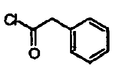 | Ex. 1 |
| 9 | 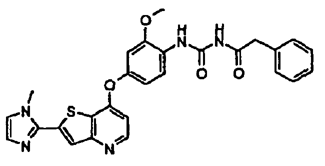 | 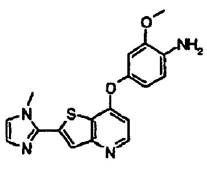 | 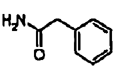 | Ex. 2 |
| 10 | 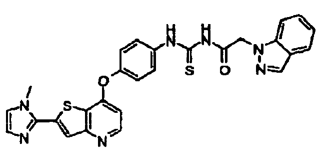 | 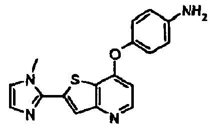 | 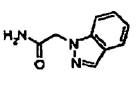 | Ex. 3 |
| 11 | 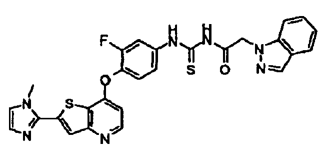 | 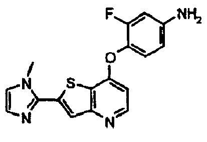 | 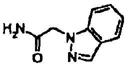 | Ex. 3 |
| 12 | 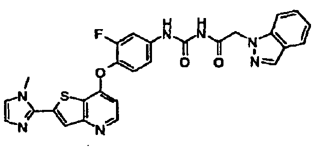 | 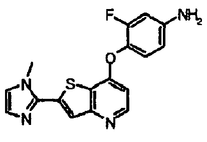 | 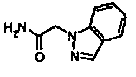 | Ex. 2 |
| 13 | 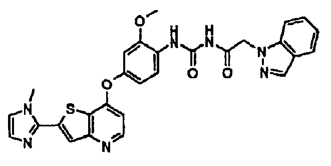 | 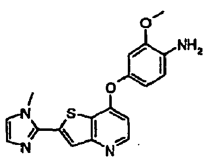 | 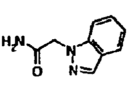 | Ex. 2 |

Data for identifying the properties of the compounds of Examples 4 to 13 are as follows.

### Example 4:

### N-[2-(4-Fluorophenyl)acetyl]-N-(4-{[2-(1-methyl-1H-2-imidazol yl)thieno[3, 2-b]pyridin-7-yl]oxy}phenyl)thiourea

¹H-NMR (CDCl₃, 400 MHz): δ 3.74 (s, 2H), 3.96 (s, 3H), 6.64 (d, J = 5.4 Hz, 1H), 7.03 - 7.30 (m, 8H), 7.71 - 7.73 (m, 3H), 8.50 (d, J = 5.6 Hz, 1H), 9.48 (s, 1H), 12.41 (s, 1H)
Mass spectrometric value (m/z): 518 [M + H]⁺

### Example 5:

### N-(3-Fluoro-4-{[2-(1-methyl-1H-2-imidazolyl)thieno[3,2-b]pyri din-7-yl]oxy}phenyl)-N-[2-(4-fluorophenyl)acetyl]thiourea

¹H-NMR (CDCl₃, 400 MHz): δ 3.73 (s, 2H), 3.97 (s, 3H), 6.57 (d, J = 5.4 Hz, 1H), 7.03 (s, 1H), 7.12 (t, J = 8.5 Hz, 2H), 7.17 (d, J = 1.0 Hz, 1H), 7.27-7.31 (m, 2H), 7.40 (d, J = 8.1 Hz, 1H), 7.72 (s, 1H), 7.94 (dd, J = 2.4 Hz, 11.7 Hz, 1H), 8.51 (d, J = 5.4 Hz, 1H), 8.71 (s, 1H), 12.47 (s, 1H)
Mass spectrometric value (m/z): 558 [M + Na]⁺

### Example 6:

### N-(4-{[2-(1-Methyl-1H-2-imidazolyl)thieno[3,2-b]pyridin-7-yl]o xy}phenyl)-N-(2-phenylacetyl)thiourea

¹H-NMR (CDCl₃, 400 MHz): δ 3.76 (s, 2H), 3.96 (s, 3H), 6.64 (d, J = 5.4 Hz, 1H), 7.03 (d, J = 1.0 Hz, 1H), 7.16 - 7.45 (m, 8H), 7.71 - 7.74 (m, 3H), 8.50 (d, J = 5.4 Hz, 1H), 8.80 (s, 1H), 12.38 (s, 1H)
Mass spectrometric value (m/z): 500 [M + H]⁺

### Example 7:

### N-(3-Fluoro-4-{[2-(1-methyl-1H-2-imidazolyl)thieno[3,2-b]pyri din-7-yl]oxy}phenyl)-N-(2-phenylacetyl)thiourea

¹H-NMR (CDCl₃, 400 MHz): δ 3.76 (s, 2H), 3.97 (s, 3H), 6.57 (d, J = 5.6 Hz, 1H), 7.03 (d, J = 1.2 Hz, 1H), 7.17 (d, J = 1.2 Hz, 1H), 7.29 - 7.33 (m, 3H), 7.37 - 7.45 (m, 4H), 7.94 (dd, J = 2.4 Hz, 11.5 Hz, 1H), 8.48 (s, 1H), 8.51 (d, J = 5.6 Hz, 1H), 12.49 (s, 1H)
Mass spectrometric value (m/z): 516 [M - H]⁻

### Example 8:

### N-(2-Methoxy-4-{[2-(1-methyl-1H-2-imidazolyl)thieno[3,2-b]py ridin-7-yl]oxy}phenyl)-N-(2-phenylacetyl)thiourea

¹H-NMR (CDCl₃, 400 MHz): δ 3.75 (s, 2H), 3.91 (s, 3H), 3.97 (s, 3H), 6.66 (d, J = 5.4 Hz, 1H), 6.80 (s, 1H), 6.81 (dd, J = 2.7 Hz, 8.3 Hz, 1H), 7.03 (d, 1.2 Hz, 1H), 7.17 (d, 1.0 Hz, 1H), 7.29 - 7.33 (m, 2H), 7.36 - 7.46 (m, 3H), 7.70 (s, 1H), 8.39 (s, 1H), 8.50 (d, J = 5.4 Hz, 1H), 8.75 - 8.77 (m, 1H), 12.59 (s, 1H)
Mass spectrometric value (m/z): 528 [M - H]⁻

### Example 9:

### N-(2-Methoxy-4-{[2-(1-methyl-1H-2-imidazolyl)thieno[3, 2-b]pyridin-7-yl]oxy}phenyl)-N-(2-phenylacetyl)urea

¹H-NMR (CDCl₃, 400 MHz): δ 3.76 (s, 2H), 3.92 (s, 3H), 3.97 (s, 3H), 6.60 (d, J = 5.4 Hz, 1H), 6.77 (s, 1H), 6.78 (dd, J = 2.7 Hz, 10.5 Hz, 1H), 7.03 (d, 1.2 Hz, 1H), 7.17 (d, 1.2 Hz, 1H), 7.30 - 7.32 (m, 2H), 7.37 - 7.44 (m, 3H), 7.57 (s, 1H), 7.70 (s, 1H), 8.25 (d, J = 8.3 Hz, 1H), 8.48 (d, J = 5.4 Hz, 1H), 10.83 (s, 1H)
Mass spectrometric value (m/z): 512 [M - H]⁻

### Example 10:

### N-[2-(1H-1-Indazolyl)acetyl]-N-(4-{[2-(1-methyl-1H-2-imidazol yl)thieno[3, 2-b]pyridin-7-yl]oxy}phenyl)thiourea

¹H-NMR (CDCl₃, 400 MHz): δ 3.95 (s, 3H), 5.23 (s, 2H), 6.63 (d, J = 5.6 Hz, 1H), 7.02 (d, J = 0.7 Hz, 1H), 7.16 - 7.27 (m, 4H), 7.41 - 7.50 (m, 2H), 7.69 - 7.71 (m, 3H), 7.80 (dd, J = 1.0 Hz, 7.1 Hz, 1H), 8.20 (d, J = 0.7 Hz, 1H), 8.49 (d, J = 5.4 Hz, 1H), 9.80 (s, 1H), 12.11 (s, 1H)
Mass spectrometric value (m/z): 562 [M + Na]⁺

### Example 11:

### N-(3-Fluoro-4-{[2-(1-methyl-1H-2-imidazolyl)thieno[3, 2-b]pyridin-7-yl]oxy}phenyl)-N-[2-(1H-1-indazolyl)acetyl]thiour ea

¹H-NMR (CDCl₃, 400 MHz): δ 3.97 (s, 3H), 5.20 (s, 2H), 6.57 (d, J = 5.4 Hz, 1H), 7.03 (s, 1H), 7.17 (d, J = 1.0 Hz, 1H), 7.30 - 7.38 (m, 2H), 7.40 - 7.54 (m, 3H), 7.70 (d, J = 0.7 Hz, 1H), 7.83 (dd, J = 1.0 Hz, 8.3 Hz, 1H), 7.91 (dd, J = 2.4 Hz, 11.5 Hz, 1H), 8.25 (d, J = 0.7 Hz, 1H), 8.50 (d, J = 5.6 Hz, 1H), 9.40 (s, 1H), 12.21 (s, 1H)
Mass spectrometric value (m/z): 556 [M - H]⁻

### Example 12:

### N-(3-Fluoro-4-{[2-(1-methyl-1H-2-imidazolyl)thieno[3, 2-b]pyridin-7-yl]oxy}phenyl)-N-[2-(1H-1-indazolyl)acetyl]urea

¹H-NMR (CDCl₃, 400 MHz): δ 3.97 (s, 3H), 5.21 (s, 2H), 6.53 (d, J = 4.6 Hz, 1H), 7.03 (d, J = 1.0 Hz, 1H), 7.16 (d, J = 1.2 Hz, 1H), 7.20 - 7.30 (m, 3H), 7.42 (d, J = 8.1 Hz, 1H), 7.51 (t, J = 7.3 Hz, 1H), 7.63 - 7.67 (m, 1H), 7.70 (s, 1H), 7.83 (d, J = 8.1 Hz, 1H), 8.21 (s, 1H), 8.28 (s, 1H), 8.48 (d, J = 5.4 Hz, 1H), 10.40 (s, 1H)
Mass spectrometric value (m/z): 540 [M - H]⁻

### Example 13:

### N-[2-(1H-1-Indazolyl)acetyl]-N-(2-methoxy-4-{[2-(1-methyl-1H -2-imidazolyl)thieno[3,2-b]pyridin-7-yl]oxy}phenyl)urea

¹H-NMR (CDCl₃, 400 MHz): δ 3.93 (s, 3H), 3.97 (s, 3H), 5.21 (s, 2H), 6.60 (d, J = 5.4 Hz, 1H), 6.77 (s, 1H), 6.78 (dd, J = 2.4 Hz, 7.3 Hz, 1H), 7.03 (s, 1H), 7.16 (d, 1.0 Hz, 1H), 7.27 - 7.29 (m, 1H), 7.42 (d, J = 8.5 Hz, 1H), 7.50 (t, 8.1 Hz, 1H), 7.69 (s, 1H), 7.82 (d, J = 8.1 Hz, 1H), 8.13 (s, 1H), 8.20 - 8.23 (m, 2H), 8.48 (d, J = 5.4 Hz, 1H), 10.66 (s, 1H)
Mass spectrometric value (m/z): 552 [M - H]⁻

### Production Example A: 7-Fluoroindazole

Hydrazine monohydrate (3 ml) was added to 2,3-difluorobenzaldehyde (1.85 g), and the mixture was heated at 180°C with stirring for 10 hr. The reaction mixture was cooled to room temperature, ethyl acetate and water were added thereto, and the organic layer was separated. The organic layer was washed with saturated brine, was dried over anhydrous sodium sulfate, and the solvent was removed by evaporation under the reduced pressure. The residue was purified by chromatography on silica gel developed with chloroform/acetone to give the title compound (790 mg, yield 45%).

¹H-NMR (CD₃OD, 400 MHz): δ 7.08 - 7.12 (m, 2H), 7.56 - 7.59 (m, 1H), 8.10 (d, J = 3.4 Hz, 1H)

### Production Example B: 2-(1H-indazolyl)acetamide

Indazole (6.55 g) was dissolved in dimethylformamide (150 ml), sodium hydride (60%) (2.80 g) was added to the solution under ice cooling, and the mixture was stirred at room temperature for 30 min. The mixture was again ice-cooled, methyl bromoacetate (6.1 ml) was added thereto, and the mixture was stirred at room temperature for 3 hr. The solvent was then removed by evaporation, and the residue was purified by chromatography on silica gel developed with chloroform/ethyl acetate to give methyl 2-(1H-indazolyl)acetate (7.5 g, yield 74%). Next, methyl 2-(1H-indazolyl)acetate (7.5 g) was dissolved in methanol (75 ml), 28% aqueous ammonia (75 ml) was added to the solution, and the mixture was stirred at room temperature for 2 hr. The precipitated crystal was collected by filtration and was washed with methanol to give the title compound (5.6 g, yield 81%).

¹H-NMR (CDCl₃, 400 MHz): 5.07 (s, 2H), 5.44 (brs, 1H), 5.81 (brs, 1H), 7.21 - 7.26 (m, 1H), 7.42 - 7.49 (m, 2H), 7.78 (d, J = 8.1 Hz, 1H), 8.12 (s, 1H)

### Production Example C: 4-[(6,7-Dimethoxy-4-quinolyl)oxy]-3-fluorophenyl isothiocyanate

4-[(6,7-Dimethoxy-4-quinolyl)oxy]-3-fluoroaniline (3.1 g) was dissolved in chloroform (100 ml). Di-2-pyridyl thiocarbamate (2.6 g) was added to the solution, and the mixture was stirred at room temperature for 20 min. Water was added, and the mixture was extracted with chloroform. The organic phase was washed with saturated brine and was then dried over sodium sulfate. The solvent was removed by evaporation. The resultant crystal was washed with a mixed solution composed of ethyl acetate and hexane (1 : 1) and was dried with a vacuum pump to give the title compound (3.3g, yield 93%).

¹H-NMR (CDCl₃, 400 MHz):4.06 (s, 3H), 4.06 (s, 3H), 6.41 (d, J = 5.4 Hz, 1H), 7.10 - 7.18 (m, 2H), 7.22 - 7.27 (m, 1H), 7.44 (s, 1H), 7.53 (s, 1H), 8.52 (d, J = 5.1 Hz, 1H)

### Production Example D: 4-[(6,7-Dimethoxy-4-quinolyl)oxy]-2-methoxyphenyl isothiocyanate

The title compound (2.2g, yield 61%) was produced in the same manner as in Production Example C, except that 4-[(6,7-dimethoxy-4-quinolyl)oxy]-2-methoxyaniline was used as the starting material.

¹H-NMR (CDCl₃, 400 MHz):3.90 (s, 3H), 4.04 (s, 3H), 4.06 (s, 3H), 6.52 (d, J = 5.1 Hz, 1H), 6.71 (dd, J = 2.4, 8.5 Hz, 1H), 6.76 (d, J = 2.4 Hz, 1H), 7.17 (d, J = 8.5 Hz, 1H), 7.44 (s, 1H), 7.49 (s, 1H), 8.53 (d, J = 5.4 Hz, 1H)

### Production Example E: Phenyl N-4-[(6,7-dimethoxy-4-quinolyl)oxy]-3-fluorophenylcarbamate

4-[(6,7-Dimethoxy-4-quinolyl)oxy]-3-fluoroaniline (5 g) was dissolved in dimethylformamide (100 ml). The solution was ice-cooled, sodium hydride (60% in oil) (955 mg) was added thereto, and the mixture was stirred at room temperature for 30 min. The mixed solution was again ice-cooled, phenyl chloroformate (3 ml) was added thereto, and the mixture was stirred at room temperature for 2 hr. Water was added, and the mixture was extracted with ethyl acetate. The organic phase was washed with saturated brine and was then dried over sodium sulfate. The solvent was removed by evaporation, the resultant crystal was washed with a mixed solution composed of ethyl acetate and hexane (1 : 1) and was dried by a vacuum pump to give the title compound (3.3 g, yield 47%).

¹H-NMR (CDCl₃, 400 MHz): 4.05 (s, 3H), 4.07 (s, 3H), 6.42 (d, J = 5.1 Hz, 1H), 7.20 - 7.29 (m, 5H), 7.40 - 7.44 (m, 3H), 7.58 - 7.64 (m, 2H), 8.51 (d, J = 5.4 Hz, 1H)

### Example 14:

N-4-[(6,7-Dimethoxy-4-quinolyl)oxy]-3-fluorophenyl-N'-[2-(1H-1-i ndazolyl)acetyl]urea

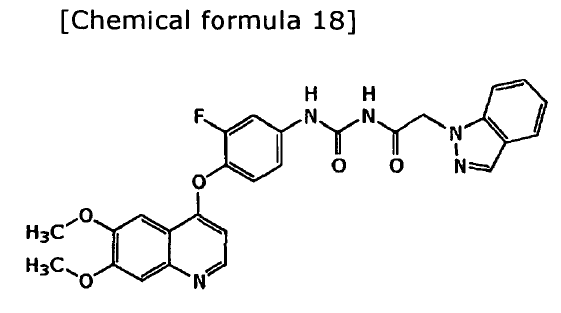

Phenyl N-4-[(6,7-dimethoxy-4-quinolyl)oxy] -3-fluorophenylcarbamate (Production Example E) (1.92 g) and 2-(1H-indazolyl)acetamide (Production Example B) (1.0 g) were added to o-xylene (150 ml), and the mixture was stirred at 180°C for 4 hr. The solvent was removed by evaporation, and the residue was purified by chromatography on silica gel developed with chloroform/methanol to give the title compound (1.0 g, yield 40%).

¹H-NMR (CDCl₃, 400 MHz): 4.05 (s, 3H), 4.06 (s, 3H), 5.21 (s, 2H), 6.40 (d, J = 5.4 Hz, 1H), 7.18 - 7.30 (m, 3H), 7.41 - 7.43 (m, 2H), 7.49 - 7.53 (m, 1H), 7.57 (s, 1H), 7.64 - 7.67 (m, 1H), 7.83 (d, J = 8.1 Hz, 1H), 8.21 (s, 1H), 8.32 (brs, 1H), 8.49 (d, J = 5.1 Hz, 1H), 10.40 (brs, 1H)
Mass spectrometric value (m/z): 514 [M⁺ - 1]

### Example 15:

N-4-[(6,7-Dimethoxy-4-quinolyl)oxy]-2-methoxyphenyl-N'-[2-(1 H-1-indazolyl)acetyl]thiourea

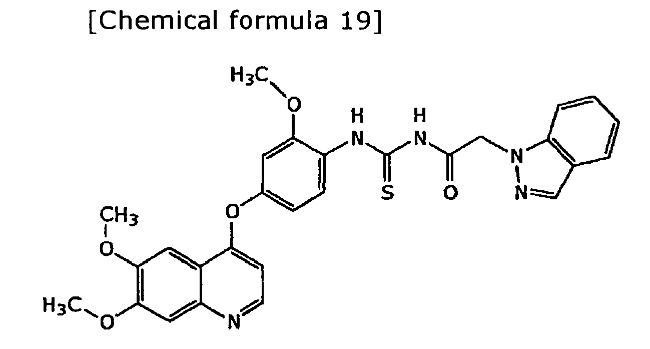

2-(1H-Indazolyl)acetamide (Production Example B) (350 mg) was dissolved in N,N-dimethylformamide (40 ml). Sodium hydride (60%) (96 mg) was added to the solution, and the mixture was stirred for 30 min. N-4-[(6,7-Dimethoxy-4-quinolyl)oxy]-2-methoxyphenyl isothiocyanate (Production Example D) (713 mg) was added thereto, and the mixture was stirred for additional 2 hr. Water was added to the mixed solution, and the mixture was extracted with ethyl acetate. The organic phase was washed with water and saturated brine and was then dried over sodium sulfate. The solvent was removed by evaporation, and acetonitrile was added to the residue for crystallization. The resultant crystal was collected by filtration, was washed with ethyl acetate and acetonitrile, and was dried with a vacuum pump to give the title compound (650 mg, yield 56%).

¹H-NMR (CDCl₃, 400 MHz): δ 3.93 (s, 3H), 4.10 (s, 3H), 4.17 (s, 3H), 5.30 (s, 2H), 6.76 - 6.86 (m, 3H), 7.28 - 7.30 (m, 1H), 7.46 - 7.53 (m, 2H), 7.62 (s, 1H), 7.83 (dd, J = 1.0, 7.3 Hz, 1H), 8.15 (s, 1H), 8.25 (d, J = 1.0 Hz, 1H), 8.57 (t, J = 6.6 Hz, 1H), 8.87 (d, J = 8.8 Hz, 1H), 9.69 (brs, 1H), 12.45 (brs, 1H)
Mass spectrometric value (m/z): 542 [M⁺ - 1]
Compounds of Examples 16 to 35 were synthesized by the methods described in Examples 14 and 15. For the compounds thus obtained, the chemical formula, starting material, synthesis method, and data for identifying the compound are shown in Table 2.

**[Table 2]**

| Example No. | Structure | Starting material A | Starting material B | Synthesis method | Mass analysis | |
|---|---|---|---|---|---|---|
| 16 | 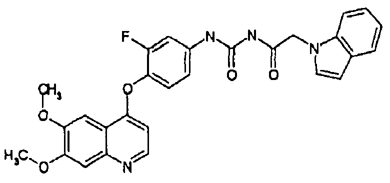 | 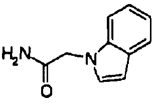 | 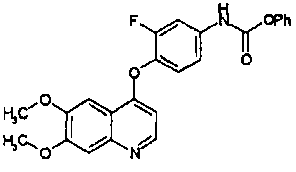 | Ex. 14 | M⁺ - 1 | 513 |
| 17 | 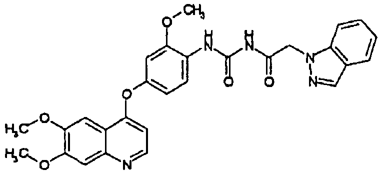 | 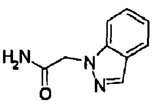 | 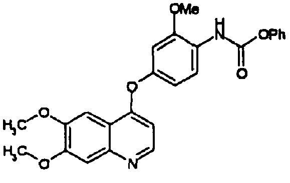 | Ex. 14 | M⁺ - 1 | 526 |
| 18 | 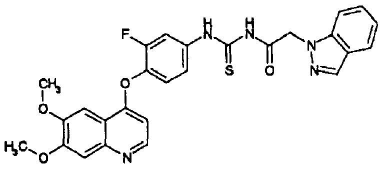 | 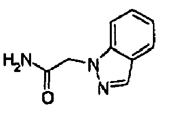 | 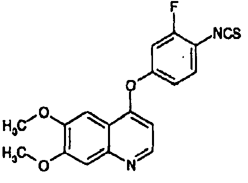 | Ex. 15 | M⁺ - 1 | 530 |
| 19 | 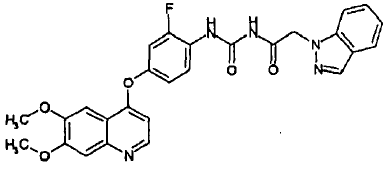 | 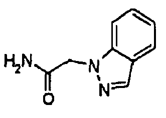 | 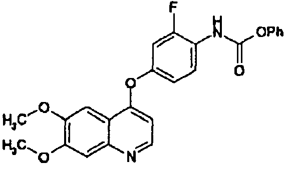 | Ex. 14 | M⁺ - 1 | 514 |
| 20 | 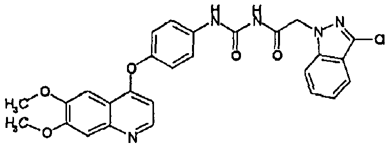 | 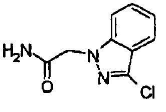 | 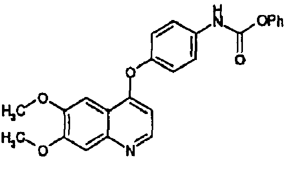 | Ex. 14 | M⁺ - 1 | 530 |
| 21 | 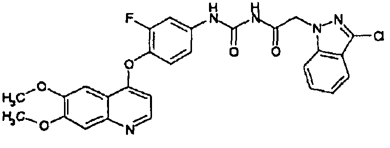 | 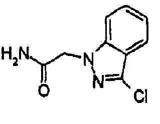 | 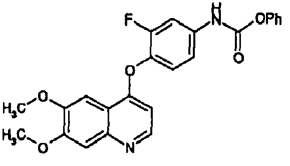 | Ex. 14 | M⁺ - 1 | 548 |
| 22 | 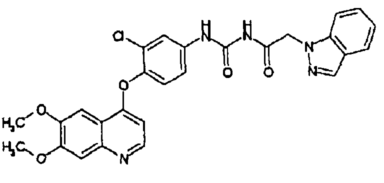 | 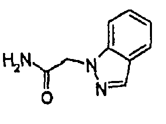 | 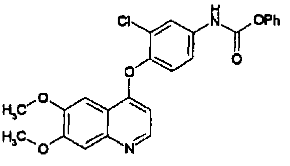 | Ex. 14 | M⁺ - 1 | 530 |
| 23 | 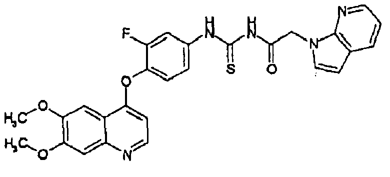 | 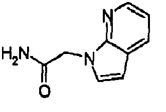 | 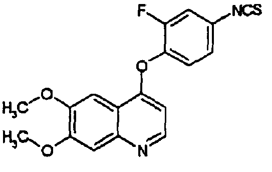 | Ex. 15 | M⁺ - 1 | 530 |
| 24 | 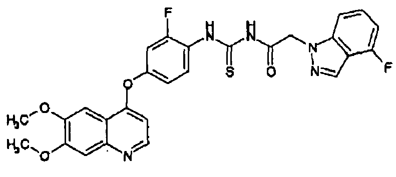 | 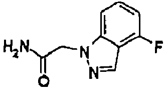 | 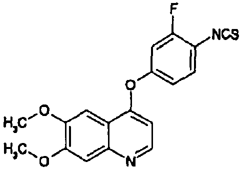 | Ex. 15 | M⁺ - 1 | 548 |
| 25 | 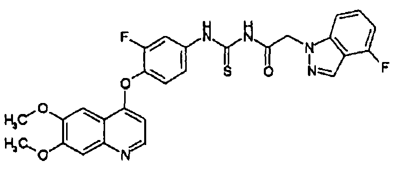 | 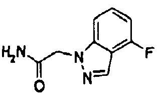 | 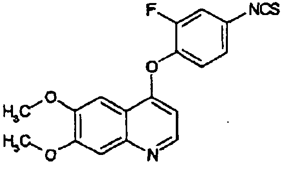 | Ex. 15 | M⁺ - 1 | 548 |
| 26 | 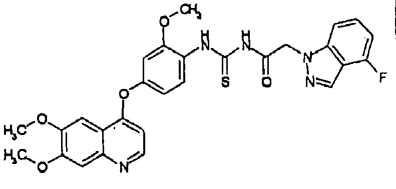 | 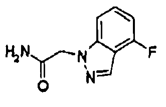 | 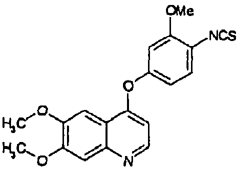 | Ex. 15 | M⁺ - 1 | 560 |
| 27 | 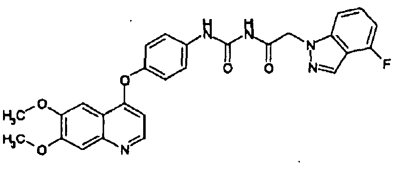 | 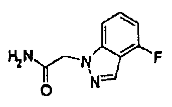 | 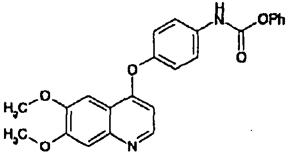 | Ex.14 | M⁺ - 1 | 514 |
| 28 | 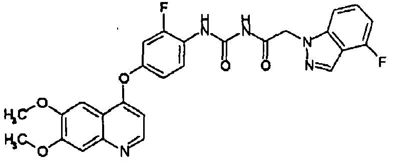 | 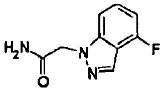 | 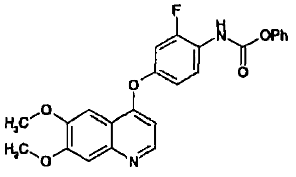 | Ex. 14 | M⁺ - 1 | 533 |
| 29 | 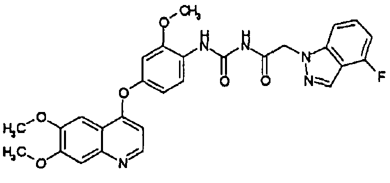 | 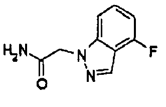 | 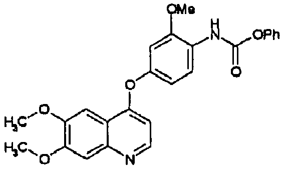 | Ex. 14 | M⁺ - 1 | 544 |
| 30 | 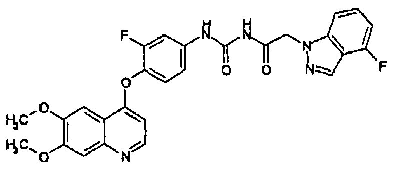 | 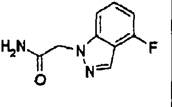 | 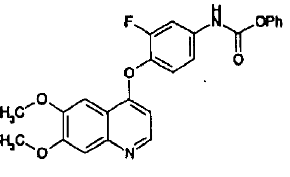 | Ex. 14 | M⁺ - 1 | 532 |
| 31 | 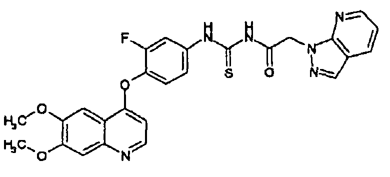 | 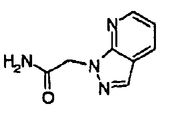 | 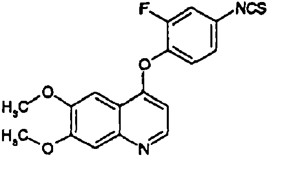 | Ex. 15 | M⁺ - 1 | 531 |
| 32 | 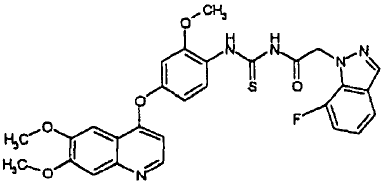 | 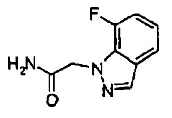 | 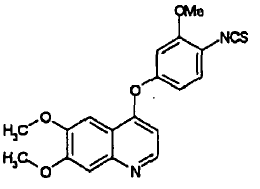 | Ex. 15 | M⁺ - 1 | 560 |
| 33 | 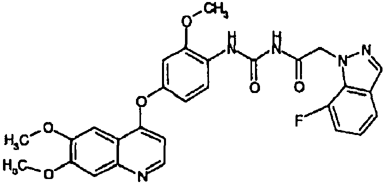 | 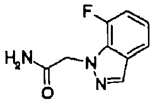 | 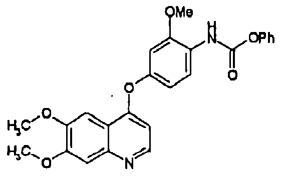 | Ex. 14 | M⁺ - 1 | 544 |
| 34 | 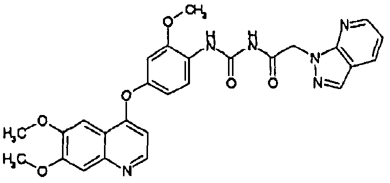 | 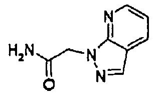 | 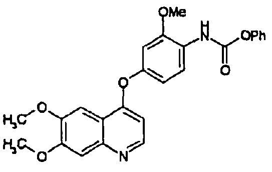 | Ex. 14 | M⁺ -1 | 527 |
| 35 | 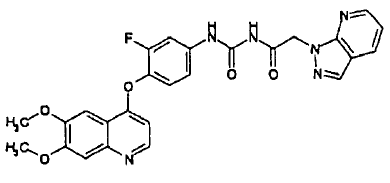 | 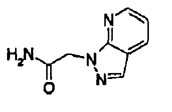 | 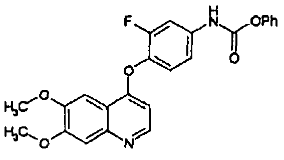 | Ex. 14 | M⁺ - 1 | 515 |

NMR data for the compounds of Examples 16 to 35 were as follows.

### Example 16:

N-4-[(6,7-Dimethoxy-4-quinolyl)oxy]-3-fluorophenyl-N'-[2-(1H-1-indolyl)acetyl]urea
¹H-NMR (CDCl₃, 400 MHz):4.05 (s, 3H), 4.05 (s, 3H), 4.97 (s, 2H), 6.40 (d, J = 5.4 Hz, 1H), 6.69 (t, J = 1.5 Hz, 1H), 7.12 (d, J = 3.2 Hz, 1H), 7.21 - 7.23 (m, 3H), 7.28 - 7.32 (m, 2H), 7.41 (s, 1H), 7.57 (s, 1H), 7.63 - 7.71 (m, 2H), 8.46 (d, J = 5.4 Hz, 1H), 10.50 (s, 1H)

### Example 17:

### N-[4-{(6,7-Dimethoxy-4-quinolyl)oxy}-2-methoxyphenyl]-N'-[2 -(1H-1-indazolyl)acetyl]urea

¹H-NMR (CDCl₃, 400 MHz): δ 3.92 (s, 3H), 4.05 (s, 3H), 4.05 (s, 3H), 5.21 (s, 2H), 6.48 (d, J = 5.4 Hz, 1H), 6.75 - 6.79 (m, 2H), 7.18 - 7.29 (m, 1H), 7.41 - 7.52 (m, 3H), 7.54 (s, 1H), 7.82 (d, J = 8.3 Hz, 1H), 8.15 (s, 1H), 8.20 - 8.23 (m, 2H), 8.48 (d, J = 5.1 Hz, 1H), 10.66 (brs, 1H)

### Example 18:

### N-[4-{(6,7-Dimethoxy-4-quinolyl)oxy}-3-fluorophenyl]-N'-[2-(1 H-1-indazolyl)acetyl]thiourea

¹H-NMR (CDCl₃, 400 MHz): δ 4.11 (s, 3H), 4.17 (s, 3H), 5.34 (s, 2H), 6.72 (d, J = 6.3 Hz, 1H), 7.29 - 7.36 (m, 2H), 7.47 - 7.55 (m, 3H), 7.64 (s, 1H), 7.83 (d, J = 8.1 Hz, 1H), 8.03 (dd, J = 2.4, 11.5 Hz, 1H), 8.15 (s, 1H), 8.25 (d, J = 1.0 Hz, 1H), 8.61 (t, J = 6.3 Hz, 1H), 9.97 (brs, 1H), 12.33 (brs, 1H)

### Example 19:

### N-[4-{(6,7-Dimethoxy-4-quinolyl)oxy}-2-fluorophenyl]-N'-[2-(1 H-1-indazolyl)acetyl]urea

¹H-NMR (CDCl₃, 400 MHz): δ 4.03 (s, 3H), 4.05 (s, 3H), 5.22 (s, 2H), 6.51 (d, J = 5.4 Hz, 1H), 6.96 - 7.01 (m, 2H), 7.23 - 7.29 (m, 1H), 7.42 - 7.53 (m, 4H), 7.83 (d, J = 8.1 Hz, 1H), 8.16 - 8.21 (m, 2H), 8.30 (brs, 1H), 8.51 (d, J = 5.1 Hz, 1H), 10.52 (brs, 1H)

### Example 20:

### N-[2-(3-Chloro-1H-1-indazolyl)acetyl]-N'-[4-{(6,7-dimethoxy-4 -quinolyl)oxy}phenyl]urea

¹H-NMR (CDCl₃, 400 MHz): δ 4.04 (s, 3H), 4.05 (s, 3H), 5.16 (s, 2H), 6.45 (d, J = 5.1 Hz, 1H), 7.16 (d, J = 8.8 Hz, 2H), 7.32 - 7.41 (m, 2H), 7.42 (s, 1H), 7.54 (s, 1H), 7.56 (s, 1H), 7.58 (d, J = 4.6 Hz, 2H), 7.78 (d, J = 8.3 Hz, 1H), 8.26 (brs, 1H), 8.48 (d, J = 5.1 Hz, 1H), 10.29 (brs, 1H)

### Example 21:

### N-[2-(3-Chloro-1H-1-indazolyl)acetyl]-N'-[4-{(6,7-dimethoxy-4 -quinolyl)oxy}-3-fluorophenyl]urea

¹H-NMR (CDCl₃, 400 MHz): δ 4.05 (s, 3H), 4.06 (s, 3H), 5.16 (s, 2H), 6.40 (d, J = 5.1 Hz, 1H), 7.21 - 7.23 (m, 2H), 7.32 - 7.43 (m, 3H), 7.54-7.59 (m, 2H), 7.64 - 7.67 (m, 1H), 7.78 (d, J = 8.3 Hz, 1H), 8.23 (brs, 1H), 8.49 (d, J = 5.1 Hz, 1H), 10.38 (brs, 1H)

### Example 22:

### N-[3-Chloro-4-{(6,7-dimethoxy-4-quinolyl)oxy}phenyl]-N'-[2-( 1H-1-indazolyl)acetyl]urea

¹H-NMR (CDCl₃, 400 MHz): δ 4.05 (s, 3H), 4.06 (s, 3H), 5.21 (s, 2H), 6.31 (d, J = 5.4 Hz, 1H), 7.20 (d, J = 8.8 Hz, 1H), 7.29 - 7.31 (m, 2H), 7.42 - 7.46 (m, 2H), 7.49 - 7.54 (m, 1H), 7.58 (s, 1H), 7.80 (s, 1H), 7.83 (d, J = 8.3 Hz, 1H), 8.22 (brs, 1H), 8.27 (s, 1H), 8.48 (d, J = 5.4 Hz, 1H), 10.37 (brs, 1H)

### Example 23:

### N-[4-{(6,7-Dimethoxy-4-quinolyl)oxy}-3-fluorophenyl]-N'-[2-(1 H-pyrrolo[2, 3-b]pyridin-1-yl)acetyl]thiourea

¹H-NMR (CDCl₃, 400 MHz): δ 4.05 (s, 3H), 4.05 (s, 3H), 5.09 (s, 2H), 6.45 (d, J = 5.4 Hz, 1H), 6.61 (dd, J = 3.7 Hz, 1H), 7.19 - 7.26 (m, 2H), 7.37 - 7.40 (m, 1H), 7.44 (s, 1H), 7.55 (s, 1H), 7.90 (dd, J = 2.4, 11.5 Hz, 1H), 8.00 (dd, J = 1.5, 7.8 Hz, 1H), 8.03 (s, 1H), 8.43 (dd, J = 1.5, 4.6 Hz, 1H), 8.51 (d, J = 5.1 Hz, 1H), 10.72 (s, 1H), 12.22 (brs, 1H)

### Example 24:

### N-[4-[{6,7-Dimethoxy-4-quinolyl}oxy]-2-fluorophenyl]-N'-[2-(4 -fluoro-1H-1-indazolyl)acetyl]thiourea

¹H-NMR (CDCl₃, 400 MHz): δ 4.06 (s, 3H), 4.06 (s, 3H), 5.20 (s, 2H), 6.45 (d, J = 5.1 Hz, 1H), 6.92 (dd, J = 7.8, 9.5 Hz, 1H), 7.21 - 7.29 (m, 2H), 7.39 - 7.49 (m, 3H), 7.55 (s, 1H), 7.90 (dd, J = 2.7, 11.5 Hz, 1H), 8.32 (brs, 1H), 8.51 (d, J = 5.4 Hz, 1H), 9.32 (s, 1H), 12.16 (brs, 1H)

### Example 25:

### N-[4-{(6,7-Dimethoxy-4-quinolyl)oxy}-3-fluorophenyl]-N'-[2-(4 -fluoro-1H-1-indazolyl)acetyl]thiourea

¹H-NMR (CDCl₃, 400 MHz): δ 4.03 (s, 3H), 4.05 (s, 3H), 5.20 (s, 2H), 6.61 (d, J = 5.1 Hz, 1H), 6.92 (dd, J = 7.8, 9.8 Hz, 1H), 6.99 - 7.03 (m, 2H), 7.22 - 7.24 (m, 1H), 7.42 - 7.52 (m, 3H), 8.30 - 8.35 (m, 2H), 8.55 (d, J = 5.4 Hz, 1H), 9.39 (s, 1H), 12.06 (brs, 1H)

### Example 26:

### N-[4-{(6,7-Dimethoxy-4-quinolyl)oxy}-2-methoxyphenyl]-N'-[2 -(4-fluoro-1H-1-indazolyl)acetyl]thiourea

¹H-NMR (CDCl₃, 400 MHz): δ 3.89 (s, 3H), 4.04 (s, 3H), 4.05 (s, 3H), 5.19 (s, 2H), 6.55 (d, J = 5.1 Hz, 1H), 6.78 - 6.82 (m, 2H), 6.91 (dd, J = 7.8, 9.8 Hz, 1H), 7.21 (d, J = 8.5 Hz, 1H), 7.42 - 7.47 (m, 2H), 7.52 (s, 1H), 8.31 (d, J = 0.7 Hz, 1H), 8.51 (d, J = 5.1 Hz, 1H), 8.73 (d, J = 8.5 Hz, 1H), 9.17 (s, 1H), 12.33 (brs, 1H)

### Example 27:

### N-[4-{(6,7-Dimethoxy-4-quinolyl)oxy}phenyl]-N'-[2-(4-fluoro-1 H-1-indazolyl)acetyl]urea

¹H-NMR (CDCl₃, 400 MHz): δ 4.04 (s, 3H), 4.05 (s, 3H), 5.22 (s, 2H), 6.45 (d, J = 5.4 Hz, 1H), 6.91 (dd, J = 7.8, 9.8 Hz, 1H), 7.15 (d, J = 9.0 Hz, 2H), 7.21 (d, J = 8.5 Hz, 1H), 7.42 - 7.47 (m, 2H), 7.54 (s, 1H), 7.57 (d, J = 9.0 Hz, 2H), 8.27 (d, J = 0.7 Hz, 1H), 8.36 (s, 1H), 8.48 (d, J = 5.4 Hz, 1H), 10.28 (s, 1H)

### Example 28:

### N-[4-{(6,7-Dimethoxy-4-quinolyl)oxy}-2-fluorophenyl]-N'-[2-(4 -fluoro-1H-1-indazolyl)acetyl]thiourea

¹H-NMR (CDCl₃, 400 MHz): δ 4.03 (s, 3H), 4.05 (s, 3H), 5.22 (s, 2H), 6.51 (d, J = 5.1 Hz, 1H), 6.92 (dd, J = 7.8, 9.5 Hz, 1H), 6.97 - 7.02 (m, 2H), 7.21 (d, J = 8.5 Hz, 1H), 7.43 - 7.48 (m, 3H), 8.16 - 8.21 (m, 1H), 8.29 (m, 2H), 8.52 (d, J = 5.1 Hz, 1H), 10.48 (s, 1H)

### Example 29:

### N-[4-{(6,7-Dimethoxy-4-quinolyl)oxy}-2-methoxyphenyl]-N'-[2 -(4-fluoro-1H-1-indazolyl)acetyl]urea

¹H-NMR (CDCl₃, 400 MHz): δ 3.91 (s, 3H), 4.05 (s, 3H), 4.05 (s, 3H), 5.21 (s, 2H), 6.48 (d, J = 5.4 Hz, 1H), 6.75 - 6.80 (m, 2H), 6.91 (dd, J = 7.8, 9.8 Hz, 1H), 7.20 (d, J = 8.5 Hz, 1H), 7.41 - 7.46 (m, 2H), 7.54 (s, 1H), 8.22 (s, 1H), 8.23 (d, J = 8.5 Hz, 1H), 8.27 (d, J = 1.0 Hz, 1H), 8.49 (d, J = 5.1 Hz, 1H), 10.63 (brs, 1H)

### Example 30:

### N-[4-{(6,7-Dimethoxy-4-quinolyl)oxy}-3-fluorophenyl]-N'-[2-(4 -fluoro-1H-1-indazolyl)acetyl]urea

¹H-NMR (CDCl₃, 400 MHz): δ 4.05 (s, 3H), 4.06 (s, 3H), 5.21 (s, 2H), 6.40 (d, J = 5.1 Hz, 1H), 6.92 (dd, J = 7.8, 9.5 Hz, 1H), 7.19 - 7.22 (m, 3H), 7.42 (s, 1H), 7.44 - 7.48 (m, 1H), 7.57 (s, 1H), 7.64 - 7.68 (m, 1H), 8.28 (d, J = 1.0 Hz, 1H), 8.33 (s, 1H), 8.49 (d, J = 5.1 Hz, 1H), 10.36 (brs, 1H)

### Example 31:

### N-[4-{(6,7-Dimethoxy-4-quinolyl)oxy}-3-fluorophenyl]-N'-[2-(1 H-pyrazolo[3, 4-b]pyridin-1-yl)acetyl]thiourea

¹H-NMR (CDCl₃, 400 MHz): δ 4.05 (s, 3H), 4.05 (s, 3H), 5.42 (s, 2H), 6.46 (d, J = 5.1 Hz, 1H), 7.23 - 7.28 (m, 2H), 7.37 - 7.40 (m, 1H), 7.44 (s, 1H), 7.55 (s, 1H), 7.91 (dd, J = 2.4, 11.5 Hz, 1H), 8.17 (dd, J = 1.5, 8.1 Hz, 1H), 8.22 (s, 1H), 8.51 (d, J = 5.4 Hz, 1H), 8.63 (dd, J = 1.5, 4.6 Hz, 1H), 9.91 (s, 1H), 12.26 (brs, 1H)

### Example 32:

### N-[4-{(6,7-Dimethoxy-4-guinolyl)oxy}-2-methoxyphenyl]-N'-[2 -(7-fluoro-1H-1-indazolyl)acetyl]thiourea

¹H-NMR (CDCl₃, 400 MHz): δ 3.89 (s, 3H), 4.04 (s, 3H), 4.05 (s, 3H), 5.38 (s, 2H), 6.55 (d, J = 5.1 Hz, 1H), 6.77 - 6.82 (m, 2H), 7.13 - 7.18 (m, 2H), 7.43 (s, 1H), 7.52 (s, 1H), 7.56 - 7.59 (m, 1H), 8.23 (d, J = 2.4 Hz, 1H), 8.51 (d, J = 5.4 Hz, 1H), 8.73 (d, J = 8.8 Hz, 1H), 9.07 (s, 1H), 12.37 (s, 1H)

### Example 33:

### N-[4-{(6,7-Dimethoxy-4-quinolyl)oxy}-2-methoxyphenyl]-N'-[2 -(7-fluoro-1H-1-indazolyl)acetyl]urea

¹H-NMR (CDCl₃, 400 MHz): δ 3.90 (s, 3H), 4.05 (s, 3H), 4.05 (s, 3H), 5.39 (s, 2H), 6.48 (d, J = 5.1 Hz, 1H), 6.75 - 6.80 (m, 2H), 7.14 - 7.18 (m, 2H), 7.42 (s, 1H), 7.55 (s, 1H), 7.57 (d, J = 8.8 Hz, 1H), 8.05 (s, 1H), 8.19 (d, J = 2.2 Hz, 1H), 8.24 (d, J = 8.5 Hz, 1H), 8.49 (d, J = 5.4 Hz, 1H), 10.67 (s, 1H)

### Example 34:

### N-[4-{(6,7-Dimethoxy-4-quinolyl)oxy}-2-methoxyphenyl]-N'-[2 -(1H-1-pyrazolo[3, 4-b]pyridin-1-yl)acetyl]urea

¹H-NMR (CDCl₃, 400 MHz): δ 3.49 (s, 3H), 4.05 (s, 3H), 4.05 (s, 3H), 5.40 (s, 2H), 6.48 (d, J = 5.4 Hz, 1H), 6.74 - 6.79 (m, 2H), 7.23 - 7.27 (m, 1H), 7.42 (s, 1H), 7.55 (s, 1H), 8.16 (dd, J = 1.5, 8.1 Hz, 1H), 8.20 (s, 1H), 8.23 (d, J = 8.8 Hz, 1H), 8.49 (d, J = 5.4 Hz, 1H), 8.52 (s, 1H), 8.62 (dd, J = 1.5, 4.6 Hz, 1H), 10.69 (brs, 1H)

### Example 35:

### N-[4-{(6,7-Dimethoxy-4-quinolyl)oxy}-3-fluorophenyl]-N'-[2-(1 H-1-pyrazolo[3, 4-b]pyridin-1-yl)acetyl]urea

¹H-NMR (CDCl₃, 400 MHz): δ 4.05 (s, 3H), 4.06 (s, 3H), 5.40 (s, 2H), 6.40 (d, J = 5.4 Hz, 1H), 7.20 - 7.29 (m, 3H), 7.42 (s, 1H), 7.57 (s, 1H), 7.65 - 7.68 (m, 1H), 8.18 (dd, J = 1.7, 8.1 Hz, 1H), 8.21 (s, 1H), 8.49 (d, J = 5.4 Hz, 2H), 8.63 (dd, J = 1.5, 4.6 Hz, 1H), 10.44 (s, 1H)

Compound A

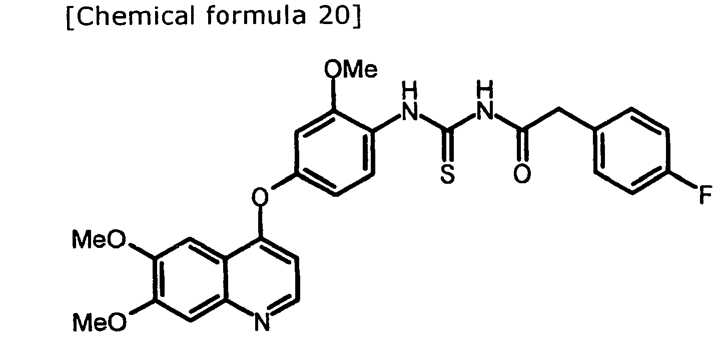

Compound A (N-[4-{(6,7-dimethoxy-4-quinolyl)oxy}-2-methoxyphenyl]-N'-[2-(4-fluorophenyl)acetyl]thi ourea) was synthesized by the synthetic method (particularly the synthetic method described in Example 2) described in Japanese Patent Laid-Open No. 158149/1999 and WO 03/00660.

¹H-NMR (CDCl₃, 400 MHz): δ 3.72 (s, 2H), 3.89 (s, 3H), 4.04 (s, 3H), 4.06 (s, 3H), 6.55 (d, J = 5.4 Hz, 1H), 6.78 - 6.82 (m, 2H), 7.12 (t, J = 8.5 Hz, 2H), 7.30 (dd, J = 5.4, 8.5 Hz, 2H), 7.43 (s, 1H), 7.52 (s, 1H), 8.43 (brs, 1H), 8.52 (d, J = 5.1 Hz, 1H), 8.75 (d, J = 8.8 Hz, 1H), 12.53 (brs, 1H)
Mass spectrometric value (m/z): 520 [M⁺ - 1]

Compound B

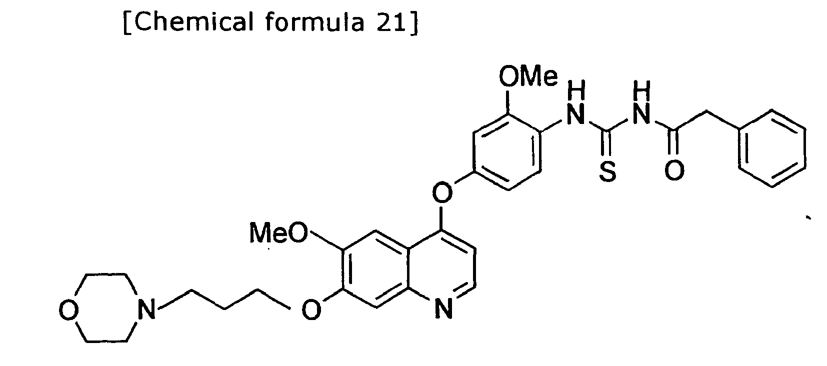

Compound B (N-{2-methoxy-4-[6-methoxy-7-(3-morpholinopropoxy)-4-quinolyl]oxyphenyl}-N'-(2 -phenylacetyl)thiourea) was synthesized by the synthetic method (particularly the synthetic method described in Example 268) described in Japanese Patent Laid-Open No. 158149/1999 and WO 03/00660.

¹H-NMR (CDCl₃, 400 MHz): δ 2.12 - 2.17 (m, 2H), 2.49 (m, 4H), 2.58 (t, J = 7.3 Hz, 2H), 3.72 (t, J = 4.4 Hz, 4H), 3.75 (s, 2H), 3.89 (s, 3H), 4.02 (s, 3H), 4.28 (t, J = 6.6 Hz, 2H), 6.54 (d, J = 5.1 Hz, 1H), 6.78 - 6.81 (m, 2H), 7.29 - 7.46 (m, 6H), 7.51 (s, 1H), 8.37 (brs, 1H), 8.50 (d, J = 5.1 Hz, 1H), 8.74 (d, J = 8.8 Hz, 1H), 12.57 (brs, 1H)

### Pharmacological Test Example 1: Measurement of human c-Met enzyme inhibitory activity

cNA coding for a part (955 to 1368aa) around human c-Met kinase domain was integrated into a pFastBac vector (Invitrogen) with a GST tag inserted therein, followed by transformation into DH10Bac to give Bacmid DNA which was then introduced into SF9 cells (GIBCO). Infection with a virus liquid was repeated five times, and SF9 cells were then broken, followed by purification using a GSH column to give a c-Met kinase active protein.

Poly Glu : Tyr = 4 : 1 (SIGMA) was coated onto a 96-well immunoplate (442404, NALGE NUNC International K.K.). Kinase buffer (50 mM HEPES (pH 7.5), 25 mM NaCl, 0.01 mM Na₃VO₄, 0.01% BSA), a test compound, and the kinase protein were added, and the mixture was incubated for 10 min. ATP (10 µM, SIGMA) was then added, and a reaction was allowed to proceed for 10 min. The plate was washed three times with PBST and was reacted with a primary antibody PY-20 (BD-610000, B.D. Bioscience) and a secondary antibody GAM (NA-9310V, Abersham) each at room temperature for one hr. The plate was washed with PBST, color development was then carried out with SUMILON (ML-1120T, Sumitomo Bakelite Co., Ltd.), and phosphorylated tyrosine was quantitatively determined by measuring the absorbance at 450 nm. IC₅₀ was calculated based on a concentration difference curve for the test compound by presuming the absorbance of the wells without the addition of ATP to be 100% inhibition and the absorbance of the wells with the addition of the medium for the test compound and ATP to be 0% inhibition.

As a result, IC₅₀ (nM) of the compounds of Examples 1, 14, and 15 were 3.2, 12.5, and 9.6, respectively. The inhibitory activity was also observed for the compounds of the other Examples.

### Pharmacological Test Example 2: Measurement (1) of inhibitory activity against c-Met-autophosphorylation using ELISA method

Human epidermoid cancer cells (A431) (available from JCRB) are cultured in an RPMI 1640 medium containing 10% fetal calf serum (purchased from GIBCO BRL) within a 5% carbon dioxide incubator until 50 to 90% confluent. The harvested cells are inoculated into wells, containing an RPMI medium containing 0.1% fetal calf serum, in a 96-well flat-bottom plate in an amount of 3 x 10⁴ per well, followed by cultivation at 37°C overnight. The medium is then replaced by a fresh RPMI medium containing 0.1% fetal calf serum. The test compound dissolved in dimethyl sulfoxide is added to each well, and the cultivation is continued at 37°C for additional one hr. A human recombinant hepatocyte growth factor (hereinafter abbreviated to "HGF") is added to a final concentration of 50 ng/ml, and the cells are stimulated at 37°C for 5 min. The medium is removed, the cells are washed with phosphate buffered physiological saline (pH 7.4), and 50 µl of a solubilization buffer (20 mM HEPES (pH 7.4), 150 mM NaCl, 0.2% Triton X-100, 10% glycerol, 5 mM sodium orthovanadylate, 5 mM disodium ethylenediaminetetraacetate, and 2 mM Na₄P₂O₇) is then added thereto. The mixture is shaken at 4°C for 2 hr to prepare a cell extract.

Separately, phosphate buffered physiological saline (50 µl, pH 7.4) containing 5 µg/ml of anti-phospho-tyrosine antibody (PY20; purchased from Transduction Laboratories) is added to a microplate for ELISA (Maxisorp; purchased from NUNC), followed by standing at 4°C overnight to form a solid phase. After washing of the plate, 300 µl of a blocking solution is added, followed by standing at room temperature for 2 hr to perform blocking. After washing, the whole quantity of the cell extract is transferred, and the plate is then allowed to stand at 4°C overnight. After washing, an anti-HGF receptor antibody (h-Met (C-12), purchased from Santa Cruz Biotechnology) is allowed to react at room temperature for one hr, and, after washing, a peroxidase-labeled anti-rabbit Ig antibody (purchased from Amersham) is allowed to react at room temperature for one hr. After washing, a chromophoric substrate for peroxidase (purchased from Sumitomo Bakelite Co., Ltd.) is added thereto to initiate a reaction. After a suitable level of color development, a reaction termination solution is added to stop the reaction, and the absorbance at 450 nm is measured with a microplate reader. The c-Met-phosphorylation inhibitory activity for each well is determined by presuming the absorbance without the addition of the test compound and with the addition of HGF to be 0% c-Met-phosphorylation inhibitory activity and the absorbance without the addition of the test compound and HGF to be 100% c-Met-phosphorylation inhibitory activity. The concentration of the test compound is varied on several levels, the inhibitory activity of c-Met-phosphorylation is determined for each case, and the concentration of the test compound necessary for inhibiting 50% of c-Met phosphorylation (IC₅₀) is calculated.

### Pharmacological Test Example 3: Measurement (2) of inhibitory activity against c-Met-autophosphorylation using ELISA method

Human gastric cancer cell line MKN45 (available from Riken) was cultured in an RPMI 1640 medium containing 10% fetal calf serum (purchased from GIBCO BRL) within a 5% carbon dioxide incubator until 50 to 90% confluent. The harvested cells were inoculated into wells, containing an RPMI medium containing 0.1% fetal calf serum, in a 96-well flat-bottom plate in an amount of 2 x 10⁴ per well, followed by cultivation at 37°C overnight. The medium was then replaced by a fresh RPMI medium containing 0.1% fetal calf serum. The test compound dissolved in dimethyl sulfoxide was added to each well, and the cultivation was continued at 37°C for additional one hr. The medium was removed, the cells were washed with phosphate buffered physiological saline (pH 7.4), and 50 µl of a solubilization buffer (20 mM HEPES (pH 7.4), 150 mM NaCl, 0.2% Triton X-100, 10% glycerol, 5 mM sodium orthovanadylate, 5 mM disodium ethylenediaminetetraacetate, and 2 mM Na₄P₂O₇) was then added thereto. The mixture was shaken at 4°C for 2 hr to prepare a cell extract.

Separately, phosphate buffered physiological saline (50 µl, pH 7.4) containing 5 µg/ml anti-phospho-tyrosine antibody (PY20; purchased from Transduction Laboratories) was added to a microplate for ELISA (Maxisorp; purchased from NUNC), followed by standing at 4°C overnight to form a solid phase. After washing of the plate, 300 µl of a blocking solution was added, followed by standing at room temperature for 2 hr to perform blocking. After washing, the whole quantity of the cell extract was transferred, and the plate was then allowed to stand at 4°C overnight. After washing, an anti-HGF receptor antibody (h-Met (C-12), purchased from Santa Cruz Biotechnology) was allowed to react at room temperature for one hr, and, after washing, a peroxidase-labeled anti-rabbit Ig antibody (purchased from Amersham) was allowed to react at room temperature for one hr. After washing, a chromophoric substrate for peroxidase (purchased from Sumitomo Bakelite Co., Ltd.) was added thereto to initiate a reaction. After a suitable level of color development, a reaction termination solution was added to stop the reaction, and the absorbance at 450 nm was measured with a microplate reader. The c-Met phosphorylation activity for each well was determined by presuming the absorbance without the addition of the test compound to be 100% c-Met phosphorylation activity and the absorbance with the addition of a largely excessive amount of positive control to be 0% c-Met phosphorylation activity. The concentration of the test compound was varied on several levels, the inhibition rate of c-Met-phosphorylation was determined for each case, and the concentration of the test compound necessary for inhibiting 50% of c-Met phosphorylation (IC₅₀) was calculated.

As a result, IC₅₀ (nM) of the compounds of Examples 1, 14, 15, and 16 were 32.8, 22.3, 13.2, and 35.9, respectively. Further, the compounds of the Examples other than these compounds were also confirmed to have inhibitory activity.

### Pharmacological Test Example 4: Tumor growth inhibitory activity against human gastric cancer cell line MKN 45

Human gastric cancer cell line MKN 45 (available from Riken) was transplanted into nude mice. When the tumor volume became about 100 mm³, the mice were grouped so that the groups each consisted of four mice and had an even average tumor volume. The test compound suspended in 0.5% methylcellulose was orally administered once a day for 9 days.

Only 0.5% methylcellulose was administered to the control group in the same manner as in the test groups. The tumor growth inhibition rate (TGIR) was calculated as follows: The tumor growth inhibition rate (TGIR) = (1 - TX/CX) x 100 wherein CX represents the volume of tumor at day X for the control group and TX represents the volume of tumor for test compound administration groups, when the tumor volume at the day of the start of the administration was presumed to be 1.

As a result, TGIR values (%) of the compounds of Examples 1 and 14 were 63.8 (9th day) and 42.6 (10th day), respectively.

### Pharmacological Test Example 5: Tumor growth inhibitory activity against human gastric cancer cell line NU-GC-4

Human gastric cancer cell line NU-GC-4 (available from Cell Resource Center for Biomedical Research, Institute of Development Aging and Cancer, Tohoku University) is transplanted into nude mice. When the tumor volume becomes about 100 mm³, the mice are grouped so that the groups each consist of four mice and have an even average tumor volume. The test compound suspended in 0.5% methylcellulose is orally administered once a day for 9 days.

Only 0.5% methylcellulose is administered to the control group in the same manner as in the test groups. The tumor growth inhibition rate (TGIR) is calculated as follows: The tumor growth inhibition rate (TGIR) = (1 - TX/CX) x 100 wherein CX represents the volume of tumor at day X for the control group and TX represents the volume of tumor for test compound administration groups, when the tumor volume at the day of the start of the administration is presumed to be 1.

As a result, TGIR (%) for compound A (25 mg/kg) was 78 (10th day).

### Pharmacological Test Example 6: Tumor growth inhibitory activity against various human tumor cell lines

Human brain tumor cell line U87-MG (ATCC), human pancreatic carcinoma cell line KP4 (Riken), human renal cancer cell line Caki-1 (available from Cell Resource Center for Biomedical Research, Institute of Development Aging and Cancer, Tohoku University), or human lung cancer cell line (LC6) (available from Central Laboratories for Experimental Animals) is transplanted into nude mice. When the tumor volume becomes about 100 mm³, the mice were divided into groups so that the groups each consist of four mice and have an even average tumor volume. The test compound suspended in 0.5% methylcellulose is orally administered once a day for 9 days. Only 0.5% methylcellulose is administered to the control group in the same manner as in the test groups. The tumor growth inhibition rate (TGIR) is calculated as follows: The tumor growth inhibition rate (TGIR) = (1 - TX/CX) x 100 wherein CX represents the volume of tumor at day X for the control group and TX represents the volume of tumor for test compound administration groups, when the tumor volume at the day of the start of the administration is presumed to be 1.

### Pharmacological Test Example 7: Measurement of c-Met and KDR enzyme inhibitory activity

c-Met protein (upstate, Lot: 25735AU, catalog code 14-526) was diluted with EDB (20 mM MOPS (pH 7.0), 1 mM EDTA, 0.01% Brij 35, 5% glycerol, 0.1% β-mercaptoethanol, and 1 mg/ml BSA) to 10 ng/µl. The KDR protein (812-1346AA) was prepared from SF21 insect cells (affinity purification, 12.4 µg/µl) according to the protocol of Invitrogen (Bac to Bac (tradename) baculovirus expression system) and was diluted with EDB to 248 ng/µl. A five-fold concentration of a reaction buffer (40 mM MOPS (pH 7.4), 1 mM EDTA) (5 µl), a ten-fold concentration of compound A (5 µl), 250 mM MnCl₂ (2.5 µl), sterilized distilled water (2.5 µl), c-Met protein or KDR protein (5 µl) was mixed thereinto under ice cooling. An ATP reaction solution (25 mM ATP, 10 µci/µl γ-³²P-ATP (1 µl), 25 mM Mg (OAc)₂, and 1.25 mM Hepes (pH7.4)) (10 µl) was added, and the mixture was allowed to react at 37°C for 10 min. A tris-SDS β-ME sample treatment liquid (301780, Daiichi Kagaku Inc.) (10 µl) was added to stop the reaction. The reaction solution was boiled at 95°C for 5 min and was then subjected to SDS-PAGE. The gel after the migration was packed, and an imaging plate (Fuji Film) was applied to the gel for transfer, followed by analysis with Typhoon (Amersham Biosciences). The IC50 value was calculated with a software for analysis Graph Pad Prism Ver.4 (Graph Pad Software, Inc.).

As a result, the IC50 was 15.5 nM for c-Met protein and was not less than 1000 nM for KDR protein (Table 3), demonstrating that compound A had high c-Met protein inhibitory activity and has low KDR protein inhibitory activity.

**[Table 3]**

| Receptor | IC50 (nM) |
|---|---|
| c-Met | 15.5 |
| KDR | > 1000 |

### Pharmacological Test Example 8: Measurement of enzyme inhibitory activity of c-Met and KDR in culture cells

MKN45 (human poorly differentiated gastric cancer cell line, Riken) and A431 (human epithelioid cancer cell line, JCRB) were used for the measurement of c-Met autophosphorylation inhibition, and HUVEC (human vascular endothelial cells, KURABO INDUSTRIES LTD.) was used for the measurement of KDR autophosphorylation inhibitory activity.

### (1) Measurement of c-Met autophosphorylation inhibition

MKN45 (2.5 x 10⁵/well) or A431 (8.5 x 10⁵/well) was inoculated into wells, containing RPMI (GIBCO) containing 0.1% fetal calf serum (JRH Biosciences), in a 6-well plate (Collagen Type I coated Plate, 4810-010, IWAKI), and, 24 hr after the inoculation, the medium was replaced with an RPMI medium containing 0.1% fetal calf serum. Thereafter, compound A was added, followed by incubation for 90 min under conditions of 37°C and 5% CO₂. The culture was removed without stimulation for MKN45 and after stimulation for 10 min with 50 ng/ml HGF (294-HG, R&D SYSTEMS) for A431. A solubilizing buffer solution (20 mM HEPES (pH 7.4), 150 mM NaCl, 0.2% TritonX-100, 10% glycerol, 5 mM sodium orthovanadylate, 5 mM disodium ethylenediaminetetraacetate, and 2 mM Na₄P₂O₇) (500 µl/well) was added to satisfactorily solubilize the cells. Immunoprecipitation (Protein G Sepharose (tradename) 4 Fast Flow, Amersham Biosciences) was carried out with a c-Met antibody (C12, Santa Culz). Treatment by SDS-PAGE and phosphorylated tyrosine antibody PY20 (610000, Amersham Biosciences) for blotting and ECL (tradename) (RPN2106, Amersham Biosciences) treatment were carried out. The film was quantified by Scion Image, and the IC50 value was determined with an analysis software Graph Pad Prism Ver.4 (Graph Pad Software, Inc.).

### (2) Measurement of KDR autophosphorylation inhibition

HUVEC was inoculated into an EGM medium (KURABO INDUSTRIES LTD.) so that the number of cells was 2 x 10⁶/100-mm Petri dish (Collagen coated Dish, 4020-010, IWAKI), and, 24 hr after the inoculation, the EGM medium was replace with an EBM medium containing 0.5% fetal calf serum (KURABO INDUSTRIES LTD.). Compound A was added, and the mixture was incubated for 90 min. After stimulation with 50 ng/ml of VEGF (100-20, PeproTech, Inc.) for 5 min, the culture was removed. The residue was subjected to immunoprecipitation with a KDR antibody (Flk-1, C-1158, Santa Cruz) by the above c-Met autophosphorylation inhibitory activity measuring method, followed by SDS-PAGE to measure KDR autophosphorylation inhibitory activity.

As a result, the IC50 of c-Met was 11.3 nM for MKN45 and 9.3 nM for A431 (Table 4). Further, the IC50 value of KDR was not less than 1000 nM in HUVEC (Table 4). The above results show that compound A have high c-Met autophosphorylation inhibitory activity but has low KDR autophosphorylation inhibitory activity, suggesting that compound A has high c-Met/KDR selectivity.

**[Table 4]**

| Receptor (cells) | IC50 (nM) |
|---|---|
| c-Met (MKN45) | 11.3 |
| c-Met (A431) | 9.3 |
| KDR (HUVEC) | > 1000 |

### Pharmacological Test Example 9: Influence on mouse lower limb bone growth plate

Nude mice (BALB-nu/nu, female, four weeks old, Charles River Japan, Inc.) were preliminarily raised for one week, and the mice were grouped so that each group consisted of 8 mice. Compound A (50 mg/kg) suspended in 0.5% methylcellulose was orally administered once a day for 4 weeks to the compound A group. On the other hand, 0.5% methylcellulose was administered to the control group in the same manner as in the compound A group.

On the 29th day after the initiation of the administration, the animals were killed from ether anesthesia. A lower limb bone around the knee region was removed, was fixed in buffer formalin, was deashed and was then subjected to paraffin embedding. A thin sliced sample was dyed with HE, and the knee bone was photographed, and whether or not thickening of the bone growth plate occurs was quantitatively determined with an image analysis software (WinROOF Ver5, MITANI CORPORATION). The average of bone growth plate sectional area ± SD in the thin sliced sample is shown in Fig. 1.

As a result, among mice in the individual groups, there was no significant difference in bone growth plate area, and the administration of compound A did not induce the thickening of the bone growth plate (Fig. 1).

## Claims

1. A compound represented by formula (I) or a pharmaceutically acceptable salt thereof, or a solvate thereof: 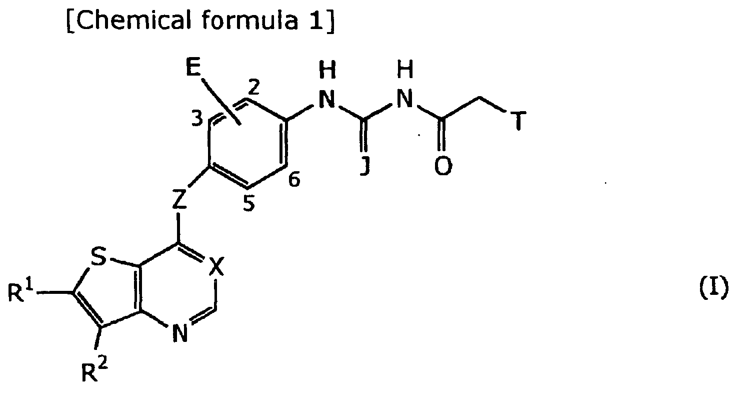 wherein
R¹ represents a hydrogen atom or an unsaturated five- or six-membered heterocyclic group optionally substituted by C₁₋₄ alkyl, C₁₋₄ alkoxy, or a halogen atom,
R² represents a hydrogen atom,
X represents CH or N,
Z represents O or S,
E is absent or represents a substituent on the phenylene group selected from a halogen atom, C₁₋₄ alkyl, and C₁₋₄ alkoxy and the numerals represent substitutable positions,
J represents S or O, and
T represents phenyl optionally substituted by a halogen atom, C₁₋₄ alkyl, or C₁₋₄ alkoxy; an unsaturated five- or six-membered heterocyclic group optionally substituted by a halogen atom, C₁₋₄ alkyl, or C₁₋₄ alkoxy; or an unsaturated nine- or ten-membered bicyclic carbocyclic or heterocyclic group optionally substituted by a halogen atom, C₁₋₄ alkyl, or C₁₋₄ alkoxy.

2. The compound according to claim 1, wherein R¹ represents a hydrogen atom or an unsaturated five-membered heterocyclic group optionally substituted by C₁₋₄ alkyl.

3. The compound according to claim 1, wherein R¹ represents a hydrogen atom or imidazolyl optionally substituted by C₁₋₄ alkyl.

4. The compound according to claim 1, wherein R¹ represents a hydrogen atom or 1H-imidazol-2-yl optionally substituted by C₁₋₄ alkyl.

5. The compound according to claim 1, wherein R¹ represents a hydrogen atom or formula (a): 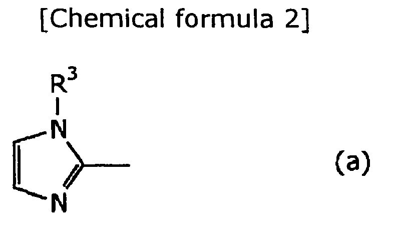 wherein R³ represents a hydrogen atom or C₁₋₄ alkyl.

6. The compound according to any one of claims 1 to 5, wherein X represents CH.

7. The compound according to any one of claims 1 to 6, wherein Z represents O.

8. The compound according to any one of claims 1 to 7, wherein E is absent or represents C₁₋₄ alkoxy at the 2-position or a halogen atom at the 3-position.

9. The compound according to any one of claims 1 to 8, wherein J represents S.

10. The compound according to any one of claims 1 to 8, wherein J represents O.

11. The compound according to any one of claims 1 to 10, wherein T represents
phenyl optionally substituted by a halogen atom, C₁₋₄ alkyl, or C₁₋₄ alkoxy;
thienyl optionally substituted by a halogen atom, C₁₋₄ alkyl, or C₁₋₄ alkoxy; or
indazolyl optionally substituted by a halogen atom, C₁₋₄ alkyl, or C₁₋₄ alkoxy.

12. The compound according to claim 1, wherein
R¹ represents formula (a) wherein R³ represents a hydrogen atom or C₁₋₄ alkyl,
R² represents a hydrogen atom,
X represents CH,
Z represents O,
E is absent or represents a substituent on the phenylene group selected from a halogen atom, C₁₋₄ alkyl, and C₁₋₄ alkoxy and the numerals represent substitutable positions,
J represents S or O,
T represents
phenyl optionally substituted by a halogen atom, C₁₋₄ alkyl, or C₁₋₄ alkoxy;
an unsaturated five-membered heterocyclic group optionally substituted by a halogen atom, C₁₋₄ alkyl, or C₁₋₄ alkoxy; or
an unsaturated nine-membered bicyclic heterocyclic group optionally substituted by a halogen atom, C₁₋₄ alkyl, or C₁₋₄ alkoxy.

13. The compound according to claim 12, wherein R³ represents C₁₋₄ alkyl.

14. The compound according to claim 12 or 13, wherein E represents C₁₋₄ alkoxy at the 2-position.

15. The compound according to claim 14, wherein E represents methoxy at the 2-position.

16. The compound according to claim 12 or 13, wherein E represents a halogen atom at the 3-position.

17. The compound according to claim 16, wherein E represents a chlorine atom at the 3-position.

18. The compound according to any one of claims 12 to 17, wherein J represents S.

19. The compound according to any one of claims 12 to 17, wherein J represents O.

20. The compound according to any one of claims 12 to 19, wherein T represents
phenyl optionally substituted by a halogen atom, C₁₋₄ alkyl, or C₁₋₄ alkoxy;
thienyl optionally substituted by a halogen atom, C₁₋₄ alkyl, or C₁₋₄ alkoxy; or
indazolyl optionally substituted by a halogen atom, C₁₋₄ alkyl, or C₁₋₄ alkoxy.

21. The compound according to any one of claims 12 to 19, wherein T represents phenyl optionally substituted by a halogen atom, C₁₋₄ alkyl, or C₁₋₄ alkoxy.

22. The compound according to any one of claims 12 to 19, wherein T represents phenyl optionally substituted by a halogen atom.

23. The compound according to any one of claims 12 to 19, wherein T represents indazolyl optionally substituted by a halogen atom, C₁₋₄ alkyl, or C₁₋₄ alkoxy.

24. The compound according to claim 1, which is selected from the following compounds:
(1)N-(3-chloro-4-{[2-(1-methyl-1H-2-imidazolyl)thieno[3,2-b]pyri din-7-yl]oxy}phenyl)-N-[2-(4-fluorophenyl)acetyl]thiourea;
(2)N-(3-fluoro-4-{[2-(1-methyl-1H-2-imidazolyl)thieno[3,2-b]pyrid in-7-yl]oxy}phenyl)-N-(2-phenylacetyl)urea;
(3)N-[2-(1H-1-indazolyl)acetyl]-N-(2-methoxy-4-{[2-(1-methyl-1H -2-imidazolyl)thieno[3,2-b]pyridin-7-yl]oxy}phenyl)thiourea;
(4)N-[2-(4-fluorophenyl)acetyl]-N-(4-{[2-(1-methyl-1H-2-imidazol yl)thieno[3,2-b]pyridin-7-yl]oxy}phenyl)thiourea;
(5)N-(3-fluoro-4-{[2-(1-methyl-1H-2-imidazolyl)thieno[3,2-b]pyrid in-7-yl]oxy}phenyl)-N-[2-(4-fluorophenyl)acetyl]thiourea;
(6)N-(4-{[2-(1-methyl-1H-2-imidazolyl)thieno[3,2-b]pyridin-7-yl]o xy}phenyl)-N-(2-phenylacetyl)thiourea;
(7)N-(3-fluoro-4-{[2-(1-methyl-1H-2-imidazolyl)thieno[3,2-b]pyrid in-7-yl]oxy}phenyl)-N-(2-phenylacetyl)thiourea;
(8)N-(2-methoxy-4-{[2-(1-methyl-1H-2-imidazolyl)thieno[3,2-b]p yridin-7-yl]oxy}phenyl)-N-(2-phenylacetyl)thiourea;
(9)N-(2-methoxy-4-{[2-(1-methyl-1H-2-imidazolyl)thieno[3,2-b]p yridin-7-yl]oxy}phenyl)-N-(2-phenylacetyl)urea;
(10)N-[2-(1H-1-indazolyl)acetyl]-N-(4-{[2-(1-methy)-1H-2-imidaz olyl)thieno[3,2-b]pyridin-7-yl]oxy}phenyl)thiourea;
(11)N-(3-fluoro-4-{[2-(1-methyl-1H-2-imidazolyl)thieno[3,2-b]pyri din-7-yl]oxy}phenyl)-N-[2-(1H-1-indazolyl)acetyl]thiourea;
(12)N-(3-fluoro-4-{[2-(1-methyl-1H-2-imidazolyl)thieno[3,2-b]pyri din-7-yl]oxy}phenyl)-N-[2-(1H-1-indazolyl)acetyl]urea; and
(13)N-[2-(1H-1-indazolyl)acetyl]-N-(2-methoxy-4-{[2-(1-methyl-1 H-2-imidazolyl)thieno[3,2-b]pyridin-7-yl]oxy}phenyl)urea.

25. A compound represented by formula (II) or a pharmaceutically acceptable salt thereof, or a solvate thereof: 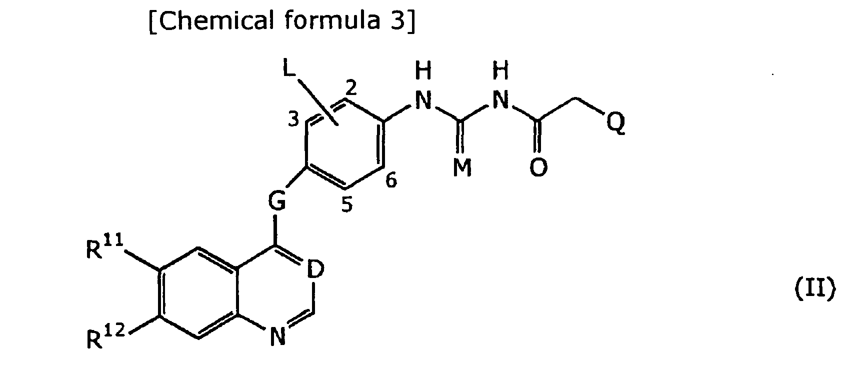 wherein
R¹¹ and R¹², which may be the same or different, represents C₁₋₄ alkoxy,
D represents CH or N,
G represents O or S,
L is absent or represents a substituent on the phenylene group selected from a halogen atom, C₁₋₄ alkyl, and C₁₋₄ alkoxy and the numerals represent substitutable positions,
M represents O or S, and
Q represents a bicyclic nine-membered unsaturated heterocyclic group optionally substituted by a halogen atom.

26. The compound according to claim 25, wherein R¹¹ and R¹² represent methoxy.

27. The compound according to claim 25 or 26, wherein D represents CH.

28. The compound according to any one of claims 25 to 27, wherein G represents O.

29. The compound according to any one of claims 25 to 28, wherein L is absent or repersents a halogen atom at the 3-positioin, a halogen atom at the 2-position, or C₁₋₄ alkoxy at the 2-position.

30. The compound according to any one of claims 25 to 29, wherein M represents O.

31. The compound according to any one of claims 25 to 29, wherein M represents S.

32. The compound according to any one of claims 25 to 31, wherein
Q represents formula (b): 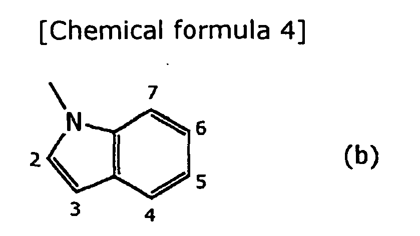 wherein the group represented by formula (b) is optionally substituted by a halogen atom and numerals represent substitutable positions;
formula (c): 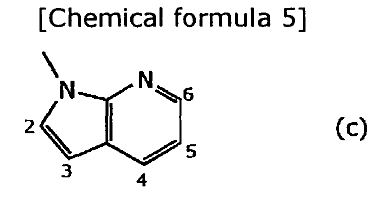 wherein the group represented by formula (c) is optionally substituted by a halogen atom and numerals represent substitutable positions;
formula (d): 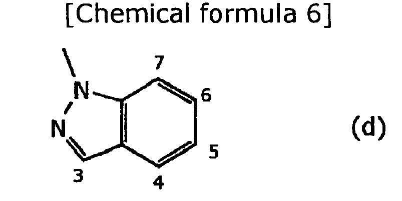 wherein the group represented by formula (d) is optionally substituted by a halogen atom and numerals represent substitutable positions; or
formula (e): 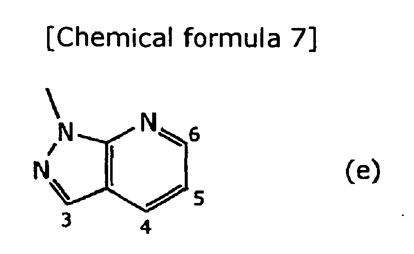 wherein the group represented by formula (e) is optionally substituted by a halogen atom and numerals represent substitutable positions.

33. The compound according to claim 25, wherein
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (b) wherein the group represented by formula (b) is optionally substituted by a halogen atom and the numerals represent substitutable positions.

34. The compound according to claim 33, wherein L represents a halogen atom at the 3-position.

35. The compound according to claim 33 or 34, wherein M represents O.

36. The compound according to any one of claims 33 to 35, wherein formula (b) is unsubstituted.

37. The compound according to claim 25, wherein
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (c) wherein the group represented by formula (c) is optionally substituted by a halogen atom and the numerals represent substitutable positions.

38. The compound according to claim 37, wherein L represents a halogen atom at the 3-position.

39. The compound according to claim 37 or 38, wherein M represents S.

40. The compound according to any one of claims 37 to 39, wherein formula (c) is unsubstituted.

41. The compound according to claim 25, wherein
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (d) wherein the group represented by formula (d) is optionally substituted by a halogen atom and the numerals represent substitutable positions.

42. The compound according to claim 41, wherein L represents a halogen atom at the 3-position.

43. The compound according to claim 41, wherein L represebnts C₁₋₄ alkoxy at the 2-position.

44. The compound according to claim 41, wherein L represents a halogen atom at the 2-position.

45. The compound according to any one of claims 41 to 44, wherein M represents O.

46. The compound according to any one of claims 41 to 44, wherein M represents S.

47. The compound according to any one of claims 41 to 46, wherein formula (d) is unsubstituted or has, as a substituent, a halogen atom at the 3-position, a halogen atom at the 4-position, or a halogen atom at the 7-position.

48. The compound according to claim 25, wherein
R¹¹ and R¹² represent methoxy,
D represents CH,
G represents O,
L is absent or represents a substituent on the phenylene group selected from a halogen atom, C₁₋₄ alkyl, and C₁₋₄ alkoxy and the numerals represent substitutable positions,
M represents O or S, and
Q represents formula (e) wherein the group represented by formula (e) is optionally substituted by a halogen atom and the numerals represent substitutable positions.

49. The compound according to claim 48, wherein L represents a halogen atom at the 3-positioin.

50. The compound according to claim 48, wherein L represents C₁₋₄ alkoxy at the 2-position.

51. The compound according to any one of claims 48 to 50, wherein M represents O.

52. The compound according to any one of claims 48 to 50, wherein M represents S.

53. The compound according to any one of claims 48 to 52, wherein formula (e) is unsubstituted.

54. The compound according to claim 25, which is selected from the following compounds:
(14)N-[4-{(6,7-dimethoxy-4-quinolyl)oxy}-3-fluorophenyl]-N'-[2-( 1H-1-indazolyl)acetyl]urea;
(15)N-[4-{(6,7-dimethoxy-4-quinolyl)oxy}-2-methoxyphenyl]-N'-[ 2-(1H-1-indazolyl)acetyl]thiourea;
(16)N-[4-{(6,7-dimethoxy-4-quinolyl)oxy}-3-fluorophenyl]-N'-[2-( 1H-1-indolyl)acetyl]urea;
(17)N-[4-{(6,7-dimethoxy-4-quinolyl)oxy}-2-methoxyphenyl]-N'-[ 2-(1H-1-indazolyl)acetyl]urea;
(18)N-[4-{(6,7-dimethoxy-4-quinolyl)oxy}-3-fluorophenyl]-N'-[2-( 1H-1-indazolyl)acetyl]thiourea;
(19)N-[4-{(6,7-dimethoxy-4-quinolyl)oxy}-2-fluorophenyl]-N'-[2-( 1H-1-indazolyl)acetyl]urea;
(20)N-[2-(3-chloro-1H-1-indazolyl)acetyl]-N'-[4-{(6,7-dimethoxy-4-quinolyl)oxy}phenyl]urea;
(21)N-[2-(3-chloro-1H-1-indazolyl)acetyl]-N'-[4-{(6,7-dimethoxy-4-quinolyl)oxy}-3-fluorophenyl]urea;
(22)N-[3-chloro-4-{(6,7-dimethoxy-4-quinolyl)oxy}phenyl]-N'-[2-( 1H-1-indazolyl)acetyl]urea;
(23)N-[4-{(6,7-dimethoxy-4-quinolyl)oxy}-3-fluorophenyl]-N'-[2-( 1H-pyrrolo[2,3-b]pyridin-1-yl)acetyl]thiourea;
(24)N-[4-[{6,7-dimethoxy-4-quinolyl}oxy]-2-fluorophenyl]-N'-[2-( 4-fluoro-1H-1-indazolyl)acetyl]thiourea;
(25)N-[4-{(6,7-dimethoxy-4-quinolyl)oxy}-3-fluorophenyl]-N'-[2-( 4-fluoro-1H-1-indazolyl)acetyl]thiourea;
(26)N-[4-{(6,7-dimethoxy-4-quinolyl)oxy}-2-methoxyphenyl]-N'-[ 2-(4-fluoro-1H-1-indazolyl)acetyl]thiourea;
(27)N-[4-{(6,7-dimethoxy-4-quinolyl)oxy}phenyl]-N'-[2-(4-fluoro-1H-1-indazolyl)acetyl]urea;
(28)N-[4-{(6,7-dimethoxy-4-quinolyl)oxy}-2-fluorophenyl]-N'-[2-( 4-fluoro-1H-1-indazolyl)acetyl]thiourea;
(29)N-[4-{(6,7-dimethoxy-4-quinolyl)oxy}-2-methoxyphenyl]-N'-[ 2-(4-fluoro-1H-1-indazolyl)acetyl]urea;
(30)N-[4-{(6,7-dimethoxy-4-quinolyl)oxy}-3-fluorophenyl]-N'-[2-( 4-fluoro-1H-1-indazolyl)acetyl]urea;
(31)N-[4-{(6,7-dimethoxy-4-quinolyl)oxy}-3-fluorophenyl]-N'-[2-( 1H-pyrazolo[3,4-b]pyridin-1-yl)acetyl]thiourea;
(32)N-[4-{(6,7-dimethoxy-4-quinolyl)oxy}-2-methoxyphenyl]-N'-[ 2-(7-fluoro-1H-1-indazolyl)acetyl]thiourea;
(33)N-[4-{(6,7-dimethoxy-4-quinolyl)oxy}-2-methoxyphenyl]-N'-[ 2-(7-fluoro-1H-1-indazolyl)acetyl]urea;
(34)N-[4-{(6,7-dimethoxy-4-quinolyl)oxy}-2-methoxyphenyl]-N'-[ 2-(1H-1-pyrazolo[3,4-b]pyridin-1-yl)acetyl]urea; and
(35)N-[4-{(6,7-dimethoxy-4-quinolyl)oxy}-3-fluorophenyl]-N'-[2-( 1H-1-pyrazolo[3,4-b]pyridin-1-yl)acetyl]urea.

55. N-[4-{(6,7-dimethoxy-4-quinolyl)oxy}-2-methoxyphenyl]-N'-[2-(4-fluorophenyl)acetyl]thiourea or a pharmaceutically acceptable salt the reof, or a solvate thereof.

56. N-{2-methoxy-4-[6-methoxy-7-(3-morpholinopropoxy)-4-quinolyl]oxyphenyl}-N'-(2-phenylacetyl)thiourea or a pharmaceuti cally acceptable salt thereof, or a solvate thereof.

57. A pharmaceutical composition comprising the compound according to any one of claims 1 to 56 or a pharmaceutically acceptable salt thereof, or a solvate thereof.

58. The pharmaceutical composition according to claim 57, for the treatment of a malignant tumor.

59. The pharmaceutical composition according to claim 58, wherein said malignant tumor is selected from the group consisting of gastric cancer, cerebral tumor, colon cancer, pancreatic cancer, lung cancer, renal cancer, ovarian cancer, and prostatic cancer.

60. Use of the compound according to any one of claims 1 to 56 or a pharmaceutically acceptable salt thereof, or a solvate thereof, for the manufacture of a medicament in the treatment of a malignant tumor.

61. The use according to claim 60, wherein said malignant tumor is selected from the group consisting of gastric cancer, cerebral tumor, colon cancer, pancreatic cancer, lung cancer, renal cancer, ovarian cancer, and prostatic cancer.

62. A method for treating a malignant tumor, comprising the step of administering a therapeutically effective amount of a compound according to any one of claims 1 to 56 or a pharmaceutically acceptable salt thereof, or a solvate thereof to a mammal in need of treatment.

63. The method according to claim 62, wherein said malignant tumor is selected from the group consisting of gastric cancer, cerebral tumor, colon cancer, pancreatic cancer, lung cancer, renal cancer, ovarian cancer, and prostatic cancer.

64. A c-Met autophosphorylation inhibitor comprising a compound according to any one of claims 1 to 56 or a pharmaceuticaly acceptable salt thereof, or a solvate thereof.

65. An agent for researching HGF/c-Met signal transduction, comprising a compound according to any one of claims 1 to 56 or a pharmaceutically acceptable salt thereof, or a solvate thereof.
